# EUROPÄISCHE PATENTSCHRIFT

(11) **EP 4 276 845 B1**
(45) Veröffentlichungstag und Bekanntmachung des Hinweises auf die Patenterteilung: **18.11.2026**
(21) Anmeldenummer: 22172818.1
(22) Anmeldetag: 11.05.2022
(51) Int. Cl.: G16H 30/40, G16H 50/20

(54) **VERFAHREN ZUM BEREITSTELLEN EINES BEARBEITETEN MEDIZINISCHEN BILDES**
METHOD FOR PROVIDING A PROCESSED MEDICAL IMAGE
PROCÉDÉ DE FOURNITURE D'UNE IMAGE MÉDICALE TRAITÉE

(43) Veröffentlichungstag der Anmeldung: 15.11.2023
(73) Patentinhaber: Siemens Healthineers AG, 91301 Forchheim (DE)
(72) Erfinder: Nolte, Björn, 90765 Fürth (DE); Schaller, Volker, 91080 Uttenreuth (DE)
(74) Vertreter: Siemens Healthineers Patent Attorneys

(56) Entgegenhaltungen:
- DE-A1- 102020 211 843

## Beschreibung

Die vorliegende Erfindung betrifft ein computerimplementiertes Verfahren zum Bereitstellen eines bearbeiteten medizinischen Bildes. Die Erfindung betrifft ferner ein System, welches ausgebildet ist, das Verfahren auszuführen.

Es ist bekannt, zum Erstellen von einer medizinischen Diagnose oder im Zuge einer medizinischen Behandlung bzw. Intervention ein medizinisches Bild eines Patienten zu erstellen und auszuwerten. Das medizinische Bild kann dabei beispielsweise ein zweidimensionales Röntgenbild, ein Ultraschallbild, ein Computer-Tomographie (Akronym: CT) Bild, ein Magnet-Resonanz-Tomographie (Akronym: MRT) Bild, ein Positronen-Emissions-Tomographie (Akronym: PET) Bild oder ein Einzel-Photonen-Emissions-Computer-Tomographie (engl. single photon emission computed tomography, Akronym: SPECT) Bild sein. Ein solches medizinisches Bild wird typischerweise von einem Radiologen ausgewertet. Das Auswerten des medizinischen Bildes umfasst dabei insbesondere ein Erstellen einer Diagnose und/oder eine Befundung und/oder ein Erstellen eines Behandlungsplans etc. Das Auswerten des medizinischen Bildes ist typischerweise sehr zeitaufwendig, insbesondere, wenn dreidimensionale Bilddaten wie ein CT-Bild oder ein MRT-Bild oder ein PET-Bild oder ein SPECT-Bild ausgewertet werden sollen. Typischerweise muss ein Radiologe zusätzlich zu dem medizinischen Bild eine Vielzahl von Patienteninformationen beim Auswerten des medizinischen Bildes berücksichtigen. Die Patienteninformationen betreffen dabei den Patienten, der auf dem medizinischen Bild abgebildet ist. Die Patienteninformationen können dabei beispielsweise in Form einer Patientenakte, insbesondere einer elektronischen Patientenakte, bereitgestellt werden. Die Patienteninformationen können beispielweise einen bereits bestehenden Befund und/oder eine Vermutung zu einem Befund und/oder ein weiteres medizinisches Bild etc. umfassen.

In der DE 10 2020 211 843 A1 ist ein computerimplementiertes Verfahren zur Analyse von Histopathologiebilddaten mittels eines Bildverarbeitungsalgorithmus beschrieben, der Ähnlichkeitsinformationen anhand eines Vergleichs von zwei Histopathologiebilddaten bereitstellen kann.

In manchen Fällen ist es außerdem notwendig, dass ein erfahrener und/oder spezialisierter zweiter Radiologe für die Auswertung des medizinischen Bildes herangezogen werden muss.

Das Auswerten des medizinischen Bildes ist somit typischerweise ein zeit- und kostenintensiver Prozess.

Es ist daher die Aufgabe der vorliegenden Erfindung, ein Verfahren bereitzustellen, welches einen Radiologen beim Auswerten eines medizinischen Bildes unterstützt.

Die Aufgabe wird gelöst durch ein Verfahren zum Bereitstellen eines bearbeiteten medizinischen Bildes, durch ein System zum Bereitstellen eines medizinischen Bildes, durch ein Computerprogrammprodukt und durch ein computerlesbares Speichermedium gemäß den unabhängigen Ansprüchen. Vorteilhafte Weiterbildungen sind in den abhängigen Ansprüchen und in der folgenden Beschreibung aufgeführt.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung betrifft ein computerimplementiertes Verfahren zum Bereitstellen eines bearbeiteten medizinischen Bildes. Das Verfahren umfasst einen Verfahrensschritt eines Empfangens eines medizinischen Bildes, wobei das medizinische Bild einen Untersuchungsbereich eines Patienten abbildet. Das medizinische Bild ist ein zweidimensionales Röntgenbild, ein Ultraschallbild, ein Computer-Tomographie (CT) Bild, ein Magnet-Resonanz-Tomographie (MRT) Bild, ein Positronen-Emissions-Tomographie (PET) Bild oder ein Einzel-Photonen-Emissions-Computer-Tomographie (SPECT) Bild. Das Verfahren umfasst außerdem einen Verfahrensschritt eines Empfangens von Patienteninformationen des Patienten. Das Verfahren umfasst außerdem einen Verfahrensschritt eines Bestimmens eines anatomischen Bereiches basierend auf dem medizinischen Bild und den Patienteninformationen. Dabei umfasst der Untersuchungsbereich den anatomischen Bereich. Der anatomische Bereich bezieht sich zudem auf eine Organstruktur oder ein Körperteil. Das Verfahren umfasst außerdem einen Verfahrensschritt eines Auswählens eines Werkzeuges basierend auf dem anatomischen Bereich. Dabei ist das Werkzeug zum Bearbeiten des in dem medizinischen Bild abgebildeten anatomischen Bereichs ausgebildet. Das Verfahren umfasst außerdem einen Verfahrensschritt eines Anwendens des ausgewählten Werkzeuges auf das medizinische Bild, wobei das bearbeitete medizinische Bild bestimmt wird. Das Verfahren umfasst außerdem einen Verfahrensschritt eines Bereitstellens des bearbeiteten medizinischen Bildes.

Das medizinische Bild wird dabei mit einer Schnittstelle empfangen. Mit anderen Worten ist die Schnittstelle zum Empfangen des medizinischen Bildes ausgebildet. Das medizinische Bild kann ein dreidimensionales oder ein zweidimensionales medizinisches Bild des Untersuchungsbereiches des Patienten sein. Das medizinische Bild kann dabei beispielsweise ein zweidimensionales Röntgenbild, ein Ultraschallbild, ein Computer-Tomographie (Akronym: CT) Bild, ein Magnet-Resonanz-Tomographie (Akronym: MRT) Bild, ein Positronen-Emissions-Tomographie (Akronym: PET) Bild oder ein Einzel-Photonen-Emissions-Computer-Tomographie (engl. single photon emission computed tomography, Akronym: SPECT) Bild sein.

Der Patient ist dabei insbesondere ein Mensch. Alternativ kann der Patient ein Tier oder ein Gegenstand sein.

Der Untersuchungsbereich umfasst dabei wenigstens einen Teil des Patienten.

Die Schnittstelle ist außerdem zum Empfangen der Patienteninformationen ausgebildet. Die Patienteninformationen sind dabei Informationen bezüglich des Patienten. Die Patienteninformationen können dabei allgemeingültige Informationen, beispielsweise physische Merkmale, zu dem Patienten und/oder Informationen aus einer älteren Untersuchung des Patienten und/oder Informationen aus eine parallel durchgeführten Untersuchung und/oder Informationen aus einer Anamnese des Patienten etc. umfassen. Außerdem können die Patienteninformationen in Ausführungen der Erfindung eine Information darüber umfassen, warum das medizinische Bild erfasst bzw. aufgenommen wurde. Mit anderen Worten können die Patienteninformationen eine Befundungsaufgabe umfassen.

In dem Verfahrensschritt des Bestimmens des anatomischen Bereiches wird der anatomische Bereich basierend auf dem medizinischen Bild und den Patienteninformationen bestimmt. Dabei wird der anatomische Bereich mit einer Recheneinheit bestimmt. Mit anderen Worten ist die Recheneinheit zum Bestimmen des anatomischen Bereiches ausgebildet. Der in dem medizinischen Bild abgebildete Untersuchungsbereich umfasst dabei den anatomischen Bereich. Der anatomische Bereich ist dabei der Bereich des Patienten, der für die Auswertung des medizinischen Bildes relevant ist. Insbesondere kann der anatomische Bereich ein Bereich des Patienten sein, der für eine Diagnose bzw. Befundung und/oder für eine Behandlung des Patienten relevant ist. Der anatomische Bereich kann dabei beispielsweise eine Läsion oder eine Akkumulation von Läsionen oder ein Bereich umfassend eine oder mehrere Läsionen sein.

Alternativ kann der anatomische Bereich sich auf eine Organstruktur bzw. auf einen Körperteil beziehen, der einen auffälligen Bereich bzw. eine auffällige Struktur umfasst. Beispielsweise kann der anatomische Bereich der linke obere Lungenlappen oder der rechte Oberschenkelknochen oder eine Mitralklappe etc. sein.

In dem Verfahrensschritt des Auswählens des Werkzeuges wird basierend auf dem anatomischen Bereich ein Werkzeug zum Bearbeiten des medizinischen Bildes ausgewählt. Dabei ist insbesondere die Recheneinheit zum Auswählen des Werkzeuges ausgebildet. Das Werkzeug kann dabei insbesondere ein Algorithmus bzw. ein Bildbearbeitungsalgorithmus sein, der auf das medizinische Bild anwendbar ist. Insbesondere kann das Werkzeug eine trainierte Funktion umfassen, die auf das medizinische Bild anwendbar ist.

Das Werkzeug ist dabei insbesondere für den anatomischen Bereich ausgebildet. Mit anderen Worten ist das Werkzeug dazu ausgebildet, auf ein medizinisches Bild angewendet zu werden, welches den anatomischen Bereich abbildet. Mit anderen Worten ist das Werkzeug zum Bearbeiten des in dem medizinischen Bild abgebildeten anatomischen Bereich ausgebildet.

In dem Verfahrensschritt des Anwendens des ausgewählten Werkzeuges wird das Werkzeug mit der Recheneinheit auf das medizinische Bild angewendet. Mit anderen Worten ist die Recheneinheit zum Anwenden des Werkzeuges auf das medizinische Bild ausgebildet. Beim Anwenden des Werkzeuges auf das medizinische Bild wird das medizinische Bild bearbeitete. Mit anderen Worten wird durch das Anwenden des Werkzeuges auf das medizinische Bild das bearbeitete medizinische Bild bestimmt. Das Bearbeiten des medizinischen Bildes kann dabei insbesondere den anatomischen Bereich betreffen.

Das bearbeitete medizinische Bild kann dabei beispielsweise eine Kennzeichnung bzw. eine Hervorhebung einer Läsion oder eines interessanten Bereiches in dem medizinischen Bild (engl.: region of interest, Akronym: ROI) umfassen. Wobei die Läsion bzw. die ROI mit dem Werkzeug erkannt wurde. Beispielsweise kann die Läsion bzw. die ROI durch Anwenden des Werkzeuges segmentiert werden.

Alternativ oder zusätzlich kann das bearbeitete Bild ein annotiertes Bild sein. In dem annotierten Bild kann beispielsweise ein durch das Anwenden des Werkzeuges bestimmter Messwert eingezeichnet sein. Der Messwert kann beispielsweise ein Durchmesser einer Läsion etc. sein.

Alternativ oder zusätzlich kann das bearbeitete medizinische Bild wenigstens ein Schichtbild umfassen, welches eine Läsion oder eine andere auffällige Struktur des in dem medizinischen Bild abgebildeten Untersuchungsbereiches abbildet. Das bearbeitete medizinische Bild kann insbesondere dann ein Schichtbild umfassen, wenn das medizinische Bild ein dreidimensionales medizinisches Bild, insbesondere ein CT-Bild oder ein MRT-Bild oder ein PET-Bild oder ein SPECT-Bild ist. Mit anderen Worten kann dann das Werkzeug dazu ausgebildet sein, ein relevantes Schichtbild aus dem entsprechenden dreidimensionalen medizinischen Bild auszuwählen, welches zum Auswerten des medizinischen Bildes besonders geeignet ist.

In dem Verfahrensschritt des Bereitstellens des bearbeiteten medizinischen Bildes wird das medizinische Bild mittels der Schnittstelle bereitgestellt. Insbesondere kann das bearbeitete medizinische Bild einer Datenbank zum Speichern des bearbeiteten medizinischen Bildes bereitgestellt werden. Alternativ oder zusätzlich wird das bearbeitete medizinische Bild einer Anzeigeeinheit bereitgestellt, die dazu ausgebildet ist, das bearbeitete medizinische Bild anzuzeigen. Die Anzeigeeinheit kann dabei ein Bildschirm oder ein Monitor sein.

Die Erfinder haben erkannt, dass basierend auf den zu Verfügung stehenden Informationen, insbesondere basierend auf dem medizinischen Bild und den Patienteninformationen ein anatomischer Bereich in dem medizinischen Bild bestimmt werden kann, der für die Auswertung des medizinischen Bildes besonders relevant ist. Die Erfinder haben erkannt, dass basierend auf dem anatomischen Bereich ein geeignetes Werkzeug zum Bearbeiten des medizinischen Bildes ausgewählt werden kann und das medizinische Bild mit diesem Werkzeug bearbeitet werden kann. Die Erfinder haben erkannt, dass durch das Bearbeiten des medizinischen Bildes mit dem Werkzeug eine Entlastung eines Radiologen bei einer Auswertung des medizinischen Bildes bewirkt werden kann. Insbesondere kann der Radiologe dann das bearbeitete medizinische Bild auswerten. Dem Radiologen können somit die Informationen aus dem medizinischen Bild und den Patienteninformationen in einer kompakten Weise dargestellt werden, wodurch Zeit des Radiologen aber auch Kosten gespart werden können.

Nach einem Aspekt der Erfindung umfassen die Patienteninformationen Einträge aus einer Patientenakte des Patienten und/oder wenigstens ein weiteres von dem medizinischen Bild verschiedenes medizinisches Bild des Patienten und/oder eine Befundungsaufgabe zu dem medizinischen Bild.

Die Patientenakte kann insbesondere eine elektronische Patientenakte sein. Die Patienten kann dabei insbesondere in einem Krankenhausinformationssystem (engl. Hospital Information System, Akronym: HIS) hinterlegt bzw. abgelegt bzw. gespeichert sein. Die Patientenakte kann dabei insbesondere allgemeine Informationen zu dem Patienten umfassen. Die allgemeinen Informationen können beispielsweise ein Alter und/oder ein Geschlecht des Patienten umfassen. Die Patientenakte kann alternativ oder zusätzlich eine Information zu einer bereits bestehenden Diagnose bzw. Befundung des Patienten umfassen. Die Patientenakte kann insbesondere eine Krankheitsgeschichte des Patienten umfassen bzw. abbilden. Alternativ oder zusätzlich kann die Patientenakte einen Befundbericht zu dem Patienten umfassen. Insbesondere kann die Patientenakte wenigstens ein weiteres von dem medizinischen Bild verschiedenes medizinisches Bild des Patienten umfassen. Alternativ oder zusätzlich kann die Patientenakte mit dem weiteren medizinischen Bild verlinkt sein. Insbesondere kann das weitere medizinische Bild ein einem Bild-Archivierungs- und Kommunikations-System (engl. Picture Archive and Communication System, Akronym: PACS) hinterlegt sein. Das weitere medizinische Bild kann dabei vor, gleichzeitig oder nach dem medizinischen Bild erfasst worden sein.

Die Befundungsaufgabe gibt an, warum das medizinische Bild erfasst wurde. Mit anderen Worten gibt die Befundungsaufgabe einen Zweck an, zu dem das medizinische Bild erfasst wurde. Insbesondere kann die Befundungsaufgabe angeben, welche Verdachtsdiagnose mit dem medizinischen Bild bestätigt oder verworfen werden soll. Alternativ oder zusätzlich kann die Befundungsaufgabe angeben, wie das medizinische Bild erfasst werden soll. Die Befundungsaufgabe kann ebenfalls Teil der Patientenakte sein.

Die Erfinder haben erkannt, dass beim Bestimmen des bearbeiteten medizinischen Bildes eine Vielzahl von verschiedenen Information zu dem Patienten berücksichtig werden kann. Die Erfinder haben erkannt, dass aus der Vielzahl der Informationen der anatomische Bereich bestimmt werden kann, der für die Auswertung des medizinischen Bildes relevant ist. Mit anderen Worten kann basierend auf der Vielzahl von Informationen das medizinische Bild derart bearbeitet werden, dass das Auswerten des medizinischen Bildes für den Radiologen vereinfacht und beschleunigt werden kann.

Nach einem weiteren Aspekt der Erfindung umfasst das Verfahren einen Verfahrensschritt eines Bestimmens derjenigen weiteren medizinischen Bilder des Patienten aus den Patienteninformationen, die den anatomischen Bereich abbilden. Das Verfahren umfasst außerdem einen Verfahrensschritt eines Bereitstellens der bestimmten weiteren medizinischen Bilder.

Insbesondere kann dabei die Patientenakte wenigstens ein weitere medizinisches Bild des Patienten umfassen bzw. mit diesem verlinkt sein.

Dabei ist die Recheneinheit dazu ausgebildet, das weitere bzw. die weiteren medizinischen Bilder zu bestimmen, die den anatomischen Bereich abbilden. Dabei können kein, ein oder mehrere weitere medizinische Bilder bestimmt werden, die den anatomischen Bereich abbilden.

Insbesondere können dafür die in der Patientenakte umfassten bzw. mit der Patientenakte verlinkten weiteren medizinischen Bilder analysiert werden. Insbesondere kann eine Ähnlichkeit zwischen dem medizinischen Bild und den weiteren medizinischen Bildern analysiert werden. Alternativ oder zusätzlich können Metainformationen der weiteren medizinischen Bilder analysiert werden. Die Metainformationen können dabei insbesondere in einem DICOM Header der weiteren medizinischen Bilder umfasst sein.

Das bzw. die weiteren medizinischen Bilder, die den anatomischen Bereich abbilden, werden in dem Verfahrensschritt des Bereitstellens der bestimmten weiteren medizinischen Bilder mit der Schnittstelle bereitgestellt. Mit anderen Worten ist die Schnittstelle außerdem zum Bereitstellen des bzw. der weiteren medizinischen Bilder ausgebildet. Das bzw. die weiteren medizinischen Bilder können dabei insbesondere einer Anzeigeeinheit bereitgestellt werden, die zum Anzeigen des oder der medizinischen Bilder ausgebildet ist. Die Anzeigeeinheit kann dabei insbesondere ein Bildschirm bzw. ein Monitor oder ein Beamer sein.

Die Erfinder haben erkannt, dass das oder die weiteren medizinischen Bilder, die ebenfalls den anatomischen Bereich des medizinischen Bildes, abbilden, den Radiologen beim Auswerten des medizinischen Bildes unterstützen können. Beispielsweise können das oder die weiteren medizinischen Bilder gemeinsam mit dem medizinischen Bild eine Zeitreihe des anatomischen Bereiches abbilden. Mit anderen Worten können die medizinischen Bilder gemeinsam eine zeitliche Entwicklung des anatomischen Bereiches abbilden. Die Erfinder haben erkannt, dass die zeitliche Entwicklung den Radiologen dabei unterstützt, das aktuelle medizinische Bild auszuwerten und eine geeignete Diagnose bzw. Befund und/oder Behandlung von dem medizinischen Bild abzuleiten.

Nach einem weiteren Aspekt der Erfindung umfasst das Verfahren einen Verfahrensschritt eines Anwendens des ausgewählten Werkzeuges auf die bestimmten weiteren medizinischen Bilder, die den anatomischen Bereich abbilden. Das Verfahren umfasst einen weiteren Verfahrensschritt eines Bereitstellens der bearbeiteten weiteren medizinischen Bilder.

In dem Verfahrensschritt des Anwendens des ausgewählten Werkzeuges auf die bestimmten weiteren medizinischen Bilder wird das Werkzeug auf das eine oder die mehreren weiteren medizinischen Bilder angewendet, die in dem Verfahrensschritt des Bestimmens derjenigen weiteren medizinischen Bilder, die anatomischen Bereich abbilden, bestimmt wurden. Das Anwenden des Werkzeuges erfolgt dabei analog zu dem Anwenden des Werkzeuges auf das medizinische Bild.

In dem Verfahrensschritt des Bereitstellens der bearbeiteten weiteren medizinischen Bilder werden die durch das Anwenden des Werkzeuges bearbeiteten weiteren medizinischen Bilder mit der Schnittstelle bereitgestellt. Mit anderen Worten ist die Schnittstelle zum Bereitstellen der bearbeiteten weiteren medizinischen Bilder ausgebildet. Insbesondere kann dabei wenigstens ein oder mehrere bearbeitete weitere medizinische Bilder bereitgestellt werden. Dabei kann das bzw. die weiteren bearbeiteten medizinischen Bilder mit der Schnittstelle einer wie oben beschrieben ausgebildeten Anzeigeeinheit bereitgestellt werden. Die Anzeigeeinheit ist dabei zum Anzeigen des oder der bearbeiteten weiteren medizinischen Bilder ausgebildet. Das bzw. die bearbeiteten weiteren medizinischen Bilder können dabei gemeinsam mit dem bearbeiteten medizinischen Bild angezeigt bzw. bereitgestellt werden.

Die Erfinder haben erkannt, dass das Auswerten des medizinischen Bildes beschleunigt und vereinfacht werden kann, wenn die vergleichbaren weiteren medizinischen Bilder analog zu dem medizinischen Bild bereitgestellt werden. Die Erfinder haben außerdem erkannt, dass dafür die weiteren medizinischen Bilder vorteilhafterweise analog zu dem medizinischen Bild mit dem Werkzeug bearbeitet wurden. Die Erfinder haben erkannt, dass auf diese Weise eine Vergleichbarkeit zwischen den Bildern sichergestellt wird. Die Erfinder haben außerdem erkannt, dass durch das Bearbeiten bzw. Anwenden des Werkzeugs auf die weiteren medizinischen Bilder und das medizinische Bild sichergestellt werden kann, dass der Radiologe schnell die relevanten Informationen aus dem medizinischen Bild und den weiteren medizinischen Bildern erkennen und auswerten kann. Beispielsweise kann durch eine Annotation der bearbeiteten medizinischen Bilder in Form eines Durchmesser einer Läsion eine Veränderung der Läsion zwischen den weiteren bearbeiteten medizinischen Bildern und dem bearbeiteten medizinischen Bildeinfach erkannt und ausgewertet werden.

Nach einem weiteren Aspekt der Erfindung umfasst der Verfahrensschritt des Bestimmens des anatomischen Bereiches einen Verfahrensschritt eines Bestimmens eines Satzes von Observablen basierend auf dem medizinischen Bild und den Patienteninformationen. Dabei basiert das Bestimmen des anatomischen Bereiches auf dem Satz von Observablen.

Dabei ist die Recheneinheit zum Bestimmen des Satzes von Observablen ausgebildet.

Der Satz von Observablen umfasst wenigstens eine Observable. Eine Observable beschreibt dabei insbesondere eine Eigenschaft des medizinischen Bildes und/oder des Patienten und/oder eines Befundes etc.

In dem Verfahrensschritt des Bestimmens des Satzes von Observablen werden somit die Informationen bzw. Eigenschaften aus den Patienteninformationen und dem medizinischen Bild extrahiert, die zum Bestimmen des anatomischen Bereiches relevant sind.

Beispielsweise kann das medizinische Bild einen DICOM (DICOM Akronym für Digital Imaging and Communications in Medicine) Header umfassen. Der DICOM Header umfasst eine Mehrzahl von DICOM Einträgen. Ein DICOM Eintrag umfasst dabei typischerweise einen DICOM Tag und einen DICOM Wert, der dem DICOM Tag zugeordnet ist. Beispielsweise kann der abgebildete Untersuchungsbereich oder anatomische Bereich in dem DICOM Wert zu dem DICOM Tag 'body region' angegeben sein. Beim Bestimmen des Satzes von Observablen können die DICOM Einträge als Observablen bestimmt werden, die zum Bestimmen des anatomischen Bereiches relevant sind. Nicht relevante DICOM Einträge werden in dem Satz von Observablen nicht berücksichtigt.

Beim Bestimmen des Satzes von Observablen können außerdem zum Bestimmen des anatomischen Bereiches relevante Informationen aus den Patienteninformationen extrahiert werden. Beispielsweise kann eine Observable ein Stichwort aus der Befundungsaufgabe oder aus einem Befundbericht etc. sein. Beispielsweise kann eine Observable eine Vorerkrankung des Patienten angeben, die aus den Patienteninformationen abgeleitet werden kann.

Das Bestimmen des Satzes von Observablen beschreibt also ein Extrahieren von Informationen bzw. Eigenschaften aus dem medizinischen Bild und den Patienteninformationen, die für das Bestimmen des anatomischen Bereiches relevant sind.

Die Erfinder haben erkannt, dass durch das Bestimmen des Satzes von Observablen die Informationen bzw. Eigenschaften, die beim Bestimmen des anatomischen Bereiches berücksichtigt werden müssen auf die relevanten Informationen reduziert werden können. Die Erfinder haben erkannt, dass auf diese Weise das Bestimmen des anatomischen Bereiches beschleunigt werden kann. Die Erfinder haben außerdem erkannt, dass durch das Bestimmen des Satzes von Observablen das Bestimmen des anatomischen Bereiches nachvollziehbar ist. Mit anderen Worten kann anhand des Satzes von Observablen nachvollzogen werden, welche Informationen zum Bestimmen des anatomischen Bereiches berücksichtigt wurden.

Nach einem weiteren Aspekt der Erfindung ist eine Observable aus dem Satz von Observablen eine systeminterne Observable oder ein Eintrag in einem dem medizinischen Bild zugeordneten Satz von Eigenschaften oder ein Körperteil gemäß einem Befund gemäß der Patienteninformationen oder ein auffälliger Laborwert gemäß der Patienteninformationen oder ein Körperbereich, von dem gemäß den Patienteninformationen eine Gewebeprobe genommen wurde oder eine Verdachtsdiagnose oder ein Befund gemäß den Patienteninformationen oder ein Stichwort aus einer Befundungsaufgabe für das medizinische Bild.

Die systeminterne Observable basiert auf einer vorbestimmten, insbesondere standardisierten, anatomischen Ontologie. Insbesondere können ein oder mehrere der systeminternen Observablen entsprechend der vorbestimmten anatomischen Ontologie hierarchisch miteinander verknüpft sein. Insbesondere basiert die vorbestimmten anatomische Ontologie auf der "SNOMED Clinical Terms" Terminologie bzw. Ontologie und/oder der "RadLex" Terminologie bzw. Ontologie. Die Verwendung dieser an sich bekannten standardisierten Terminologien zur Definition bzw. zum Aufbau der Observablen stellt die Kompatibilität mit den klinischen Abläufen sicher, verbessert den Austausch von Informationen und erleichtert die Zuordnung des anatomischen Bereichs.

Der dem medizinischen Bild zugeordnete Satz von Eigenschaften kann insbesondere ein wie oben beschrieben ausgebildeter DICOM Header sein. Dabei beschreibt jeder DICOM Eintrag eine Eigenschaft des medizinischen Bildes oder des in dem medizinischen Bild abgebildeten Untersuchungsbereiches oder des Patienten. Die Observable kann somit wenigstens ein DICOM Eintrag oder ein entsprechender DICOM Wert sein.

Die Observable kann insbesondere ein Körperteil des Patienten sein, der in einem Befund in den Patienteninformationen erwähnt wird. Beispielsweise kann ein Befund in den Patienteninformationen, insbesondere in der Patientenakte, die Lunge des Patienten betreffen. Dann kann die Observable "Lunge" sein.

Die Observable kann alternativ ein auffälliger Laborwert gemäß den Patienteninformationen, insbesondere gemäß der Patientenakte sein. Der Laborwert kann dabei insbesondere speziell eine Eigenschaft eines Organs beschreiben. Beispielsweise kann ein erhöhter Bilirubinwert auf eine Fehlfunktion der Leber hinweisen. Die Observable kann somit "Bilirubin" sein. Beim Bestimmen des anatomischen Bereiches kann dann von der Observablen auf die Leber geschlossen werden.

Die Observable kann alternativ einen Körperteil angeben, von welchem gemäß der Patienteninformationen, insbesondere der Patientenakte, eine Gewebeprobe entnommen wurde. Die Gewebeprobe kann dabei insbesondere eine Biopsie sein. Eine Biopsie des Brustgewebes des Patienten kann darauf hindeuten, dass eine Auffälligkeit in der Brust des Patienten bekannt ist. Die Observable kann dann beispielsweise "Brust" sein.

Die Observable kann alternativ eine Verdachtsdiagnose sein. Insbesondere kann die Observable auf der Verdachtsdiagnose basieren. Insbesondere kann die Observable ein Stichwort bzw. eine Zusammenfassung der Verdachtsdiagnose sein. Beispielsweise kann die Verdachtsdiagnose "Lungenkarzinom" sein. Alternativ kann die Verdachtsdiagnose in Form einer standardisierten, anatomischen Ontologie angegeben sein. Die Observable kann dann insbesondere eine systeminterne Observable sein. Die Verdachtsdiagnose kann insbesondere von den Patienteninformationen umfasst sein. Die Verdachtsdiagnose kann von einem medizinischen Personal basierend auf weiteren Untersuchungen des Patienten erstellt worden sein.

Die Observable kann alternativ ein Befund gemäß der Patienteninformationen, insbesondere der Patientenakte, sein. Insbesondere kann die Observable ein Stichwort des Befundes sein. Alternativ kann die Observable eine systeminterne Observable sein, die den Befund beschreibt. Der Befund kann insbesondere auf weiteren Untersuchungen des Patienten basieren. Der Befund kann aus der Krankheitsgeschichte des Patienten bekannt sein.

Die Observable kann alternativ ein Stichwort aus der Befundungsaufgabe für das medizinische Bild sein. Die Befundungsaufgabe gibt an, zu welchem Zweck das medizinische Bild erfasst werden sollte. Insbesondere kann die Befundungsaufgabe angeben, wie das medizinische Bild erfasst werden soll. Insbesondere kann die Befundungsaufgabe angeben, welcher Untersuchungsbereich wie in dem medizinischen Bild abgebildet sein soll. Beispielsweise kann die Befundungsaufgabe lauten "Thorax-Aufnahme, rechter Lungenflügel". Dann kann die Observable "Thorax" oder "rechter Lungenflügel" sein.

Die Erfinder haben erkannt, dass anhand der Observablen relevante Informationen aus dem medizinischen Bild und den Patienteninformationen extrahiert werden können. Die Erfinder haben erkannt, dass eine Vielzahl von verschiedenen Informationen in dem Satz von Observablen berücksichtigt werden können.

Nach einem weiteren Aspekt der Erfindung wird in dem Verfahrensschritt des Bestimmens des anatomischen Bereiches wird der anatomische Bereich mittels Mustererkennung aus einer Mehrzahl von anatomischen Bereichen bestimmt. Dabei ist jedem der anatomischen Bereiche der Mehrzahl von anatomischen Bereichen ein Satz von typischen Observablen zugeordnet. Dabei wird mittels Mustererkennung derjenige anatomische Bereich aus der Mehrzahl von anatomischen Bereichen bestimmt, für den der zugeordnete Satz von typischen Observablen am besten mit dem basierend auf dem medizinischen Bild und den Patienteninformationen bestimmten Satz von Observablen übereinstimmt.

Insbesondere wird somit eine Mehrzahl von anatomischen Bereichen bereitgestellt, für welche im Vorfeld jeweils ein Satz von typischen Observablen bestimmt wurde. Die typischen Observablen sind dabei wie oben, bezüglich der Observablen beschrieben, ausgebildet. Der Satz von typischen Observablen eines anatomischen Bereiches umfasst insbesondere diejenigen Observablen, die im Zusammenhang mit dem anatomischen Bereich typischerweise bzw. besonders häufig vorkommen. Mit anderen Worten umfasst der Satz von typischen Observablen diejenigen Observablen, die auf den anatomischen Bereich hindeuten. Dabei kann eine Observable von mehr als einem Satz von typischen Observablen umfasst sein. Dabei kann die Kombination von typischen Observablen in einem Satz von typischen Observablen für den entsprechenden anatomischen Bereich typisch bzw. eindeutig sein.

Mittels Mustererkennung kann aus der Mehrzahl von Sätzen von typischen Observablen derjenige Satz bestimmt werden, der dem bestimmten Satz von Observablen am besten entspricht. Mit anderen Worten wird derjenige Satz von typischen Observablen bestimmt, der die größte Übereinstimmung mit dem für das medizinische Bild bestimmten Satz von Observablen aufweist. Der bestimmte Satz von Observablen ist der Satz von Observablen, der in dem Verfahrensschritt des Bestimmens des Satzes von Observablen basierend auf dem medizinischen Bild und den Patienteninformationen bestimmt wurde.

Als anatomischer Bereich wird dann derjenige anatomische Bereich aus der Mehrzahl von anatomischen Bereichen bestimmt, der dem zuvor ausgewählten Satz von typischen Observablen zugeordnet ist bzw. durch diesen beschrieben wird.

Die Mustererkennung kann dabei insbesondere auf einer trainierten Funktion basieren. Dabei wird die trainierte Funktion auf den bestimmten Satz von Observablen angewendet. Dabei wird der anatomische Bereich bestimmt, dessen Satz von typischen Observablen am besten mit dem bestimmten Satz von Observablen übereinstimmt.

Im Allgemeinen ahmt eine trainierte Funktion kognitive Funktionen nach, die Menschen mit menschlichem Denken verbinden. Insbesondere durch auf Trainingsdaten basierendem Training kann sich die trainierte Funktion an neue Umstände anpassen sowie Muster erkennen und extrapolieren.

Im Allgemeinen können Parameter einer trainierten Funktion mittels Trainings angepasst werden. Insbesondere kann dafür ein beaufsichtigtes (supervised) Training, ein halbüberwachtes (semi-supervised) Training, ein unbeaufsichtigtes (unsupervised) Training, ein verstärkendes Lernen (reinforcement learning) und/oder ein aktives Lernen (active learning) verwendet werden. Darüber hinaus kann Repräsentationslernen (ein alternativer Begriff ist "Merkmalslernen") (representation learning bzw. feature learning) verwendet werden. Insbesondere können die Parameter der trainierten Funktionen durch mehrere Trainingsschritte iterativ angepasst werden.

Insbesondere kann eine trainierte Funktion ein neuronales Netzwerk, eine Unterstützungsvektormaschine (support vector machine), einen Zufallsbaum bzw. einen Entscheidungsbaum (decision tree) und/oder ein Bayes'sches Netzwerk umfassen, und/oder die trainierte Funktion kann auf k-Mittel-Clustering (k-means clustering), Q-Learning, genetischen Algorithmen und/oder Assoziationsregeln basieren. Insbesondere kann eine trainierte Funktion eine Kombination aus mehreren unkorrelierten Entscheidungsbäumen bzw. ein Ensemble aus Entscheidungsbäumen (random forest) umfassen. Insbesondere kann die trainierte Funktion mittels XGBoosting (extreme Gradient Boosting) bestimmt werden. Insbesondere kann ein neuronales Netzwerk ein tiefes neuronales Netzwerk (deep neural network), ein Faltungs-neuronales Netzwerk (convolutional neural network) oder ein Faltungs-tiefes neuronales Netzwerk (convolutional deep neural network) sein. Darüber hinaus kann ein neuronales Netzwerk ein kontradiktorisches Netzwerk (adversarial network), ein tiefes kontradiktorisches Netzwerk (deep adversarial network) und/oder ein generatives kontradiktorisches Netzwerk (generative adversarial network) sein. Insbesondere kann ein neuronales Netzwerk ein rekurrentes neuronales Netzwerk (recurrent neural network) sein. Insbesondere kann ein rekurrentes neuronales Netzwerk ein Netzwerk mit langem Kurzzeitgedächtnis (long-short-term-memory, LSTM), insbesondere eine Gated Recurrent Unit (GRU), sein. Insbesondere kann eine trainierte Funktion eine Kombination der beschriebenen Ansätze umfassen. Insbesondere werden die hier beschriebenen Ansätze für eine trainierte Funktion Netzwerkarchitektur der trainierten Funktion genannt.

Die Erfinder haben erkannt, dass durch Mustererkennung der anatomische Bereiche bestimmt werden kann. Dafür können Sätze von typischen Observablen für verschiedene anatomische Bereiche mit dem bestimmten Satz von Observablen verglichen werden und der am besten passende Satz von typischen Observablen und damit der zugehörige anatomische Bereich bestimmt werden. Die Erfinder haben erkannt, dass dies eine effiziente und einfache Methode ist, den anatomischen Bereich zu bestimmen.

Nach einem weiteren Aspekt der Erfindung umfassen die Patienteninformationen wenigstens einen Eintrag aus einer Patientenakte und/oder eine Befundungsaufgabe. Dabei ist dem medizinischen Bild ein Satz von Eigenschaften zugeordnet. Dabei umfasst der Verfahrensschritt des Bestimmens des Satzes von Observablen einen Verfahrensschritt eines Anwendens einer linguistischen Analysefunktion auf den wenigstens einen Eintrag aus der Patientenakte und/oder die Befundungsaufgabe. Dabei wird wenigstens ein Schlagwort bestimmt. Der Verfahrensschritt des Bestimmens des Satzes von Observablen umfasst außerdem einen Verfahrensschritt eines Abgleichens des wenigstens einen Schlagwortes mit dem Satz von Eigenschaften. Dabei wird bei einem positiven Abgleich das Schlagwort in den Satz von Observablen aufgenommen.

Der Eintrag aus der Patientenakte kann beispielsweise ein Laborwert oder ein Eintrag zu einer entnommenen Gewebeprobe etc. sein. Mit anderen Worten kann ein Eintrag aus der Patientenakte eine Information zu einer Krankheitsgeschichte des Patienten umfassen. Insbesondere kann ein Eintrag aus der Patientenakte eine Information zu wenigstens einer vorhergehenden Diagnose und/oder Untersuchung und/oder Behandlung und/oder Befundung des Patienten umfassen. Alternativ oder zusätzlich kann der Eintrag in die Patientenakte eine allgemeine Information zu dem Patienten umfassen. Die allgemeine Information zu dem Patienten kann beispielsweise eine Information über das Alter oder das Geschlecht des Patienten sein. Mit anderen Worten kann die allgemeine Information eine Information über ein physisches Merkmal des Patienten umfassen.

Die Befundungsaufgabe ist wie oben beschrieben ausgebildet. Die Befundungsaufgabe gibt insbesondere an, zu welchem Zweck das medizinische Bild erfasst wurde. Die Befundungsaufgabe kann außerdem angeben, wie das medizinischen Bild erfasst werden sollte bzw. von welchem Untersuchungsbereich das medizinische Bild erfasst werden sollte.

Die linguistische Analysefunktion ist dazu ausgebildet, einen Text inhaltlich zu analysieren. Die linguistische Analysefunktion ist mit anderen Worten dazu ausgebildet, den wenigstens einen Eintrag aus der Patientenakte und/oder die Befundungsaufgabe basierend auf linguistischer Information, insbesondere natürliche Sprache enthaltender Information, als Einzelinformationen zu identifizieren und bereitzustellen. Insbesondere kann die linguistische Analysefunktion dazu ausgebildet sein, Metadaten des wenigstens einen Eintrags aus der Patientenakte, wie beispielsweise einen DICOM Header, auszuwerten.

Die linguistische Analysefunktion wird auf den wenigstens einen Eintrag aus der Patientenakte und/oder die Befundungsaufgabe angewendet. Dabei wird der Text des wenigstens einen Eintrags und/oder der Befundungsaufgabe analysiert. Basierend auf der Analyse wird wenigstens ein Schlagwort bestimmt. Insbesondere kann für jeden Eintrag aus der Patientenakte und/oder für die Befundungsaufgabe wenigstens ein Schlagwort bestimmt werden. Insbesondere kann jeweils für zusammenhängende bzw. voneinander abhängige Einträge aus der Patientenakte wenigstens ein Schlagwort bestimmt werden.

Das Schlagwort beschreibt somit wenigstens einen Eintrag aus der Patientenakte und/oder die Befundungsaufgabe. Das Schlagwort kann insbesondere eine Zusammenfassung des wenigstens einen Eintrags und/oder der Befundungsaufgabe sein.

Die Recheneinheit ist dabei zum Ausführen des Verfahrensschrittes des Anwendens der linguistischen Analysefunktion ausgebildet.

Der Satz von Eigenschaften ist wie oben beschrieben ausgebildet. Insbesondere kann der Satz von Eigenschaften ein DICOM Header sein.

In dem Verfahrensschritt des Abgleichens des wenigstens einen Schlagwortes mit dem Satz von Eigenschaften wird abgeglichen, ob der Satz von Eigenschaften das Schlagwort umfasst. Dabei ist die Recheneinheit zum Ausführen des Verfahrensschrittes des Abgleichens ausgebildet.

Bei dem Abgleich kann insbesondere überprüft werden, ob der Satz von Eigenschaften das Schlagwort exakt oder sinngemäß umfasst.

Wenn der Satz von Eigenschaften ein DICOM Header ist, wird insbesondere abgeglichen, ob ein DICOM Wert dem Schlagwort exakt oder sinngemäß entspricht.

Beispielsweise kann mittels der linguistischen Analyse bestimmt worden sein, dass ein Eintrag aus der Patientenakte die Lunge des Patienten betrifft und ein anderer Eintrag aus der Patientenakte ein Knie des Patienten betrifft. Dabei werden in dem Verfahrensschritt des Anwendens der linguistischen Analysefunktion die beiden Schlagwort "Knie" und "Lunge" bestimmt. Der Satz von Eigenschaften umfasst einen DICOM Wert "Thorax". Der in dem medizinischen Bild abgebildete Untersuchungsbereich umfasst somit den Thorax des Patienten. In dem Verfahrensschritt des Abgleichens wird zwischen "Lunge" und "Thorax" ein positiver Abgleich festgestellt, während der Abgleich zwischen "Knie" und "Thorax" negativ ist. Somit wird das Schlagwort "Lunge" in den Satz von Observablen aufgenommen. In Ausführungen der Erfindung kann alternativ oder zusätzlich "Thorax" in den Satz von Observablen aufgenommen werden.

Die Erfinder haben erkannt, dass durch den beschriebenen Abgleich sichergestellt werden kann, dass aus den Patienteninformationen und/oder der Befundungsaufgabe keine Observablen extrahiert werden, die nichts mit dem medizinischen Bild zu tun haben. Der Patient kann in seiner Krankheitsgeschichte bereits verschiedene nicht zusammenhängende Untersuchungen und Diagnosen erhalten haben, die unabhängig von der Auswertung des aktuellen medizinischen Bildes sind. Das Anwenden der linguistischen Analysefunktion und das Abgleichen verhindern dabei, dass von dem medizinischen Bild unabhängige und für die Auswertung irrelevante Observablen in den Satz von Observablen aufgenommen werden.

Nach einem weiteren Aspekt der Erfindung umfasst das Verfahren einen Verfahrensschritt eines Bereitstellens des Satzes von Observablen. Das Verfahren umfasst außerdem einen Verfahrensschritt eines Empfangens einer Nutzereingabe bezüglich des Satzes von Observablen. Dabei wird die Nutzereingabe beim Bestimmen des anatomischen Bereiches berücksichtigt.

In dem Verfahrensschritt des Bereitstellens des Satzes von Observablen wird der Satz von Observablen einem Nutzer, insbesondere dem Radiologen, mittels der Schnittstelle bereitgestellt. Insbesondere kann der Satz von Observablen dem Nutzer mit einer Anzeigeeinheit, insbesondere einem Monitor bzw. einem Bildschirm, bereitgestellt bzw. angezeigt werden. Dabei kann der Satz von Observablen dem Nutzer in Form einer Liste angezeigt werden.

In dem Verfahrensschritt des Empfangens der Nutzereingabe wird die Nutzereingabe mittels der Schnittstelle empfangen. Die Nutzereingabe kann dabei durch den Nutzer mittels der Schnittstelle bereitgestellt werden. Die Nutzereingabe kann beispielsweise über eine Eingabe über einen berührungsempfindlichen Bildschirm (engl. Touchscreen) und/oder über ein berührungsempfindliches Tastfeld (engl. Touchpad) und/oder über einen Mausklick etc. bereitgestellt werden.

In der Nutzereingabe können diejenigen Observablen aus dem Satz von Observablen ausgewählt sein, die der Nutzer für das medizinische Bild relevant hält. Alternativ oder zusätzlich kann die Nutzereingabe ein oder mehrere Observablen umfassen, die bisher in dem Satz von Observablen noch nicht umfasst waren.

Der Satz von Observablen kann dann entsprechend der Nutzereingabe angepasst werden. Insbesondere kann der Satz von Observablen auf die ausgewählten bzw. für relevant befundenen Observablen eingeschränkt werden. Alternativ oder zusätzlich kann die eine oder mehreren zusätzlichen Observablen in den Satz von Observablen aufgenommen werden. Der anatomische Bereich kann dann basierend auf diesem angepassten Satz von Observablen bestimmt werden. Mit anderen Worten wird die Nutzereingabe beim Bestimmen des anatomischen Bereiches berücksichtigt.

In einer Ausführung der Erfindung können die Verfahrensschritte des Bereitstellens des Satzes von Observablen und des Empfangens der Nutzereingabe von dem Verfahrensschritt des Bestimmens des anatomischen Bereiches umfasst sein. Insbesondere kann dann der anatomische Bereich basierend auf dem mittels der Nutzereingabe angepassten Satz von Observablen bestimmt werden.

Alternativ kann zunächst der anatomische Bereich basierend auf dem in dem Verfahrensschritt des Bestimmens des Satzes von Observablen bestimmten Satz von Observablen bestimmt werden. In dem Verfahrensschritt des Bereitstellens des Satzes von Observablen kann dann zusätzlich der bestimmte anatomische Bereich bereitgestellt werden. Die Nutzereingabe kann dann den bestimmten anatomischen Bereich berücksichtigen. Mit anderen Worten kann dann basierend auf der Nutzereingabe unter Berücksichtigung des bereits bestimmten anatomischen Bereiches der Satz von Observablen angepasst werden. Anschließend kann der anatomische Bereich erneut bestimmt werden basierend auf dem angepassten Satz von Observablen.

Die Erfinder haben erkannt, dass eine Nutzereingabe beim Bestimmen des anatomischen Bereiches berücksichtigt werden kann, indem der Satz von Observablen entsprechend der Nutzereingabe angepasst wird. Die Erfinder haben erkannt, dass auf diese Weise der Satz von Observablen kontrolliert und gegebenenfalls korrigiert bzw. angepasst werden kann. Auf diese Weise können beispielsweise fehlende Informationen aus dem medizinischen Bild und/oder den Patienteninformationen, die in dem Satz von Observablen noch nicht berücksichtigt sind, aber dem Nutzer bekannt sind, in den Satz von Observablen aufgenommen werden. Unpassende Observablen, die möglicherweise zu einem falschen anatomischen Bereich führen würden, können aus dem Satz von Observablen gelöscht werden.

Nach einem weiteren Aspekt der Erfindung umfasst die Nutzereingabe bezüglich des Satzes von Observablen ein Priorisieren und/oder ein Löschen und/oder ein Auswählen der Observablen.

Wie oben beschrieben können durch die Nutzereingabe eine oder mehrere Observablen aus dem Satz von Observablen gelöscht werden. Insbesondere werden die gelöschten Observablen beim Bestimmen des anatomischen Bereiches nicht mehr berücksichtigt.

Alternativ oder zusätzlich können wie oben beschrieben durch die Nutzereingabe eine oder mehrere Observablen aus dem Satz von Observablen ausgewählt werden, die als relevant für das medizinische Bild befunden werden. Insbesondere können dann die nicht ausgewählten Observablen des Satzes von Observablen aus dem Satz von Observablen gelöscht bzw. entfernt werden.

Alternativ oder zusätzlich können wie oben beschrieben eine oder mehrere noch nicht von dem Satz von Observablen umfasste Observablen durch die Nutzereingabe ausgewählt und zu dem Satz von Observablen hinzugefügt werden.

Alternativ oder zusätzlich können die von dem Satz von Observablen umfassten Observablen durch die Nutzereingabe priorisiert werden. Insbesondere kann ein bereits durch Auswählen oder Löschen angepasster Satz von Observablen priorisiert werden. Dabei gibt die Priorisierung an, welche Observablen besonders relevant für das medizinische Bild bzw. für die Auswertung des medizinischen Bildes sind. Insbesondere kann bei der oben beschriebenen Mustererkennung zum Bestimmen des anatomischen Bereiches die Priorisierung berücksichtigt werden. Je höher eine Observable priorisiert ist, desto wichtiger ist es bei der Mustererkennung, dass auch der Satz von typischen Observablen die entsprechende Observable umfasst.

Die Erfinder haben erkannt, dass verschiedene Formen der Nutzereingabe berücksichtigt werden können. Die Erfinder haben erkannt, dass entweder der Satz von Observablen direkt entsprechend angepasst werden kann oder beim Bestimmen des anatomischen Bereiches durch die Priorisierung entsprechend berücksichtigt werden kann.

Nach einem weiteren Aspekt der Erfindung umfasst das Verfahren einen Verfahrensschritt eines Bestimmens eines Suchprofils für ähnliche Patienten basierend auf den Patienteninformationen und/oder dem medizinischen Bild. Das Verfahren umfasst außerdem einen Verfahrensschritt eines Bestimmens von wenigstens einem ähnlichen Patienten aus mehreren Referenz-Patienten durch Anwenden des Suchprofils in einem medizinischen Informationssystem, in welchem die verschiedenen Referenz-Patienten angelegt sind. Das Verfahren umfasst außerdem einen Verfahrensschritt eines Bereitstellens einer mit dem wenigstens einen ähnlichen Patienten im Zusammenhang stehenden Information.

Der Verfahrensschritt des Bestimmens des Suchprofils wird insbesondere mit der Recheneinheit ausgeführt.

Ein ähnlicher Patient kann insbesondere bezüglich seiner allgemeinen bzw. physischen Merkmale bzw. Eigenschaften dem Patienten ähnlich sein. Alternativ oder zusätzlich kann ein ähnlicher Patienten dem Patienten bezüglich seiner Krankheitsgeschichte, insbesondere bezüglich seiner Diagnosen, Untersuchungen, Befunde etc., dem Patienten ähnlich sein. Beispielsweise kann der ähnliche Patient an einer gleichen Krankheit leiden, wie der Patient. Alternativ oder zusätzlich kann ein gleiches oder vergleichbares medizinisches Bild von dem ähnlichen Patienten erfasst und vorteilhafterweise ausgewertet worden sein.

Das Suchprofil wird basierend auf den Patienteninformationen und/oder dem medizinischen Bild bestimmt bzw. erstellt.

Das Suchprofil beschreibt dabei eine Suche nach einem Patienten, der ähnliche oder gleiche Eigenschaften hinsichtlich seiner physischen Eigenschaften bzw. Merkmale und/oder hinsichtlich seiner Krankheitsgeschichte aufweist, wie der der Patient. Mit anderen Worten gibt das Suchprofil Eigenschaften vor, die ein Patient aufweisen muss, um dem Patienten ähnlich zu sein.

Insbesondere kann zum Bestimmen des Suchprofils zunächst ein, wie oben beschrieben ausgebildeter Satz von Observablen bestimmt werden. Mit anderen Worten kann der Verfahrensschritt des Bestimmens des Suchprofils einen Verfahrensschritt eines Bestimmens eines Satzes von Observablen basierend auf den Patienteninformationen und/oder dem medizinischen Bild umfassen. Der Verfahrensschritt des Bestimmens des Satzes von Observablen kann dabei wie oben beschrieben ausgebildet sein.

Das Suchprofil kann somit basierend auf dem Satz von Observablen bestimmt werden.

In Ausführungen der Erfindung kann das Suchprofil durch Anwenden einer trainierten Funktion bestimmt werden. Die trainierte Funktion kann dabei wie oben beschrieben ausgebildet sein. Insbesondere kann die trainierte Funktion basierend auf einer Nutzerrückmeldung (engl.: Feedback) angepasst bzw. optimiert werden. Die trainierte Funktion kann mittels föderalem Lernen (eng. federated learning) trainiert bzw. gelernt werden. Dafür kann an verschiedenen Institutionen basierend auf den dort vorhandenen Referenz-Patienten jeweils eine vortrainierte Funktion bestimmt werden. Basierend auf den verschiedenen vortrainierten Funktionen kann dann die trainierte Funktion bestimmt werden, ohne dass Daten bzw. Informationen bzgl. der Referenz-Patienten die verschiedenen Institutionen verlassen müssen. Eine Institution kann dabei insbesondere ein Krankenhaus oder ein Krankenhausverbund oder eine Arztpraxis etc. sein.

Zum Trainieren der trainierten Funktion wird die trainierte Funktion auf die in einer Institution angelegten Referenz-Patienten angewendet. Mit anderen Worten wird die trainierte Funktion auf zu den Referenz-Patienten hinterlegte Referenz-Patienteninformationen und/oder medizinische Bilder angewendet. Dabei ist die trainierte Funktion dazu ausgebildet, Ähnlichkeiten zu erkennen und die Referenz-Patienten zu Clustern. Durch die Rückmeldung des Nutzers kann die trainierte Funktion derart angepasst bzw. trainiert werden, dass das Clustern besser mit der Rückmeldung übereinstimmt.

Alternativ oder zusätzlich kann das Suchprofil basierend auf dem anatomischen Bereich bestimmt werden. Dabei kann beim Bestimmen des Suchprofils berücksichtigt werden, welcher anatomische Bereich bestimmt wurde. Mit anderen Worten basiert das Suchprofil dann auf dem basierend auf den Patienteninformationen und/oder dem medizinischen Bild bestimmten anatomischen Bereich. Dabei kann das Suchprofil derart ausgebildet sein, dass mit dem Suchprofil nach einem ähnlichen Patienten gesucht werden kann, bei welchem eine Untersuchung desselben anatomischen Bereiches durchgeführt wurde bzw. von welchem in demselben anatomischen Bereich ein medizinisches Bild erfasst wurde.

Die mehreren Referenz-Patienten sind in dem medizinischen Informationssystem angelegt. Angelegt bedeutet, dass jedem Referenz-Patienten ein Patientenidentifikator zugeordnet ist, welcher auf den Referenz-Patienten betreffende Patienteninformationen und/oder medizinische Bilder und/oder andere Informationen verweist.

Das medizinische Informationssystem kann dabei insbesondere eine Datenbank sein. Das medizinische Informationssystem kann dabei insbesondere ein Bildarchivierungs-und-Kommunikationssystem (engl. Picture Archiving and Communication System, Akronym: PACS) und/oder ein Laborinformationssystem (Akronym: LIS) und/oder ein Krankenhausinformationssystem (engl. Hospital Information System, Akronym: HIS) und/oder ein Radiologieinformationssystem (Akronym: RIS) etc. sein.

Die mehreren relevanten Patienten sind dabei insbesondere die Patienten, zu denen Informationen in dem medizinischen Informationssystem hinterlegt sind.

In dem Verfahrensschritt des Bestimmens von wenigstens einem ähnlichen Patienten wird das Suchprofil mit der Recheneinheit in dem medizinischen Informationssystem angewendet. Dabei werden der oder die Referenz-Patienten der mehreren Patienten bestimmt, die das Suchprofil erfüllen. Die derart bestimmten Referenz-Patienten sind dem Patienten ähnliche Patienten. "Das Suchprofil erfüllen" bedeutet, dass ein Referenz-Patient, der das Suchprofil erfüllt, wenigstens einen bestimmten Anteil an Eigenschaften aufweist, die durch das Suchprofil vorgegeben sind. Insbesondere kann ein derart bestimmter Referenz-Patient alle durch das Suchprofil vorgegebenen Eigenschaften erfüllen bzw. aufweisen.

In dem Verfahrensschritt des Bereitstellens der mit dem wenigstens einen ähnlichen Patienten im Zusammenhang stehenden Information wird die mit dem wenigstens einen ähnlichen Patienten in Zusammenhang stehende Information mit der Schnittstelle bereitgestellt. Insbesondere kann die mit dem wenigstens einen ähnlichen Patienten in Zusammenhang stehende Information mittels einer Anzeigeeinheit bereitgestellt, insbesondere angezeigt werden.

Die mit dem wenigstens einen ähnlichen Patienten in Zusammenhang stehende Information kann dabei insbesondere von Patienteninformationen zu dem ähnlichen Patienten und/oder von einem medizinischen Bild eines Untersuchungsbereiches des ähnlichen Patienten und/oder einer Befundungsaufgabe den ähnlichen Patienten betreffend umfasst sein.

Die Erfinder haben erkannt, dass der Radiologe beim Auswerten des medizinischen Bildes unterstützt werden kann, indem eine mit wenigstens einem ähnlichen Patienten in Zusammenhang stehende Information bereitgestellt wird. Auf diese Weise kann der Radiologe beispielsweise auf Besonderheiten in dem medizinischen Bild aufmerksam gemacht werden, die bei dem ähnlichen Patienten zu einer bestimmten Diagnose bzw. zu einem Befund geführt haben. Außerdem kann dem Radiologen anhand der Information zu dem ähnlichen Patienten aufgezeigt werden, welche weiteren Behandlungen bei dem ähnlichen Patienten durchgeführt wurden und in Ausführungen der Erfindung wie erfolgreich diese Behandlungen waren.

Nach einem weiteren Aspekt der Erfindung umfasst die mit dem wenigstens einen ähnlichen Patienten in Zusammenhang stehende Information eine Diagnose des ähnlichen Patienten. Dabei umfasst der Verfahrensschritt des Bereitstellens einer mit dem ähnlichen Patienten in Zusammenhang stehenden Information einen Verfahrensschritt eines Bereitstellens der Diagnose des ähnlichen Patienten.

Die Diagnose beschreibt insbesondere eine Erkrankung, die bei dem ähnlichen Patienten diagnostiziert wurde. Insbesondere kann die Diagnose bei dem ähnlichen Patienten wenigstens teilweise auf einem medizinischen Bild beruhen, welches dem medizinischen Bild des Patienten entspricht. Entsprechen bedeutet, dass das die medizinischen Bilder mit einer gleichen Bildgebungsmodalität erfasst wurden und/oder denselben Untersuchungsbereich abbilden. Eine Bildgebungsmodalität kann dabei beispielsweise ein Röntgen oder eine Computer-Tomographie oder eine Magnet-Resonanz-Tomographie oder ein Ultraschall etc. sein.

In dem Verfahrensschritt des Bereitstellens der Diagnose wird die Diagnose des ähnlichen Patienten mittels der Schnittstelle bereitgestellt. Insbesondere kann dabei die Diagnose mit einer Anzeigeeinheit angezeigt werden.

Die Erfinder haben erkannt, dass die Auswertung des medizinischen Bildes beschleunigt und vereinfacht werden kann, wenn der Radiologe darüber informiert ist, welche Diagnose bei einem ähnlichen Patienten erstellt wurde. Auf diese Weise wird dem Radiologen eine Information darüber bereitgestellt, auf was er in dem medizinischen Bild gegebenenfalls besonders achten sollte, um eine identische oder ähnliche Diagnose für den Patienten zu bestätigen oder auszuschließen.

Nach einem weiteren Aspekt der Erfindung umfasst die mit dem ähnlichen Patienten in Zusammenhang stehende Information ein medizinisches Bild des ähnlichen Patienten. Dabei umfasst das Verfahren außerdem einen Verfahrensschritt eines Anwendens des ausgewählten Werkzeuges auf das medizinische Bild des ähnlichen Patienten. Dabei wird ein bearbeitetes medizinisches Bild des ähnlichen Patienten bestimmt. Das Verfahren umfasst außerdem einen Verfahrensschritt eines Bereitstellens des bearbeiteten medizinischen Bildes des ähnlichen Patienten.

Das medizinische Bild des ähnlichen Patienten bildet dabei einen Untersuchungsbereich des ähnlichen Patienten ab. Der in dem medizinischen Bild des ähnlichen Patienten abgebildete Untersuchungsbereich ist wenigstens teilweise identisch zu dem in dem medizinischen Bild abgebildeten Untersuchungsbereich des Patienten. Insbesondere bilden das medizinische Bild des ähnlichen Patienten und das medizinische Bild des Patienten denselben anatomischen Bereich jeweils in Bezug auf den ähnlichen Patienten und den Patienten ab. Das medizinische Bild des ähnlichen Patienten ist dabei vorteilhafterweise in der gleichen Bildmodalität wie das medizinische Bild des Patienten. Mit anderen Worten zeigen die beiden medizinischen Bilder vorteilhafterweise dieselben physikalischen Eigenschaften des abgebildeten Untersuchungsbereiches. Mit anderen Worten sind das medizinische Bild des ähnlichen Patienten und das medizinische Bild des Patienten vorteilhafterweise vergleichbar.

In dem Verfahrensschritt des Anwendens des ausgewählten Werkzeuges auf das medizinischen Bild des ähnlichen Patienten wird das ausgewählte Werkzeug mit der Recheneinheit angewendet. Dabei wird das ausgewählte Werkzeug auf das medizinische Bild des ähnlichen Patienten analog dazu angewendet, wie es auf das medizinische Bild des Patienten angewendet wird. Auf diese Weise wird das medizinische Bild des ähnlichen Patienten analog zu dem medizinischen Bild des Patienten bearbeitet.

In dem Verfahrensschritt des Bereitstellens des bearbeiteten medizinischen Bildes des ähnlichen Patienten wird das bearbeitete medizinische Bild des ähnlichen Patienten mit der Schnittstelle bereitgestellt. Insbesondere kann das bearbeitete medizinischen Bild des ähnlichen Patienten mit einer Anzeigeeinheit, insbesondere einem Bildschirm bzw. Monitor, bereitgestellt werden. Mit anderen Worten kann das bearbeitete medizinischen Bild des ähnlichen Patienten angezeigt werden.

Dabei kann das bearbeitete medizinische Bild des ähnlichen Patienten gemeinsam mit dem bearbeiteten medizinischen Bild des Patienten angezeigt werden.

Die Erfinder haben erkannt, dass das Auswerten des medizinischen Bildes beschleunigt und vereinfacht werden kann, wenn ein weiteres vergleichbares medizinisches Bild eines ähnlichen Patienten, welches analog bearbeitet wurde, als Vergleich bereitgestellt wird. Insbesondere kann der Radiologe auf diese Weise auf Bereiche in dem medizinischen Bild des ähnlichen Patienten aufmerksam gemacht werden, die für die Auswertung des medizinischen Bildes des Patienten ebenfalls relevant sein können.

Die Erfindung betrifft außerdem ein System zum Bereitstellen eines bearbeiteten medizinischen Bildes. Das System umfasst eine Schnittstelle und eine Recheneinheit. Die Schnittstelle und/oder die Recheneinheit sind zum Ausführen eines Verfahrensschrittes eines Empfangens eines medizinischen Bildes ausgebildet. Dabei bildet das medizinische Bild einen Untersuchungsbereich eines Patienten ab. Das medizinische Bild ist ein zweidimensionales Röntgenbild, ein Ultraschallbild, ein Computer-Tomographie (CT) Bild, ein Magnet-Resonanz-Tomographie (MRT) Bild, ein Positronen-Emissions-Tomographie (PET) Bild oder ein Einzel-Photonen-Emissions-Computer-Tomographie (SPECT) Bild. Die Schnittstelle und/oder die Recheneinheit sind zum Ausführen eines Verfahrensschritt eines Empfangens von Patienteninformationen des Patienten ausgebildet. Die Schnittstelle und/oder die Recheneinheit sind zum Bestimmen eines anatomischen Bereiches basierend auf dem medizinischen Bild und den Patienteninformationen ausgebildet. Dabei umfasst der Untersuchungsbereich den anatomischen Bereich. Der anatomische Bereich bezieht sich dabei auf eine Organstruktur oder ein Körperteil. Die Schnittstelle und/oder die Recheneinheit sind außerdem zum Auswählen eines Werkzeuges basierend auf dem anatomischen Bereich ausgebildet. Dabei ist das Werkzeug zum Bearbeiten des in dem medizinischen Bild abgebildeten anatomischen Bereichs ausgebildet. Die Schnittstelle und/oder die Recheneinheit sind außerdem zum Anwenden des ausgewählten Werkzeuges auf das medizinische Bild ausgebildet. Dabei wird ein bearbeitetes medizinisches Bild bestimmt. Die Schnittstelle und/oder die Recheneinheit sind außerdem zum Bereitstellen des bearbeiteten medizinischen Bildes ausgebildet.

Eine solches System kann insbesondere dazu ausgebildet sein das zuvor beschriebene Verfahren zum Bereitstellen eines bearbeiteten medizinischen Bildes und seine Aspekte auszuführen. Das System ist dazu ausgebildet dieses Verfahren und seine Aspekte auszuführen, indem die Schnittstelle und die Recheneinheit ausgebildet sind, die entsprechenden Verfahrensschritte auszuführen.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendete Systeme auf einfache Weise durch ein Software-Update nachgerüstet werden können, um auf die beschriebene Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Insbesondere betrifft die Erfindung auch ein Computerprogrammprodukt mit einem Computerprogramm, welches direkt in einen Speicher eines Systems ladbar ist, mit Programmabschnitten, um alle Schritte des oben beschriebenen Verfahrens zum Bereitstellen eines bearbeiteten medizinischen Bildes und seine Aspekte auszuführen, wenn die Programmabschnitte von dem System ausgeführt werden.

Insbesondere betrifft die Erfindung ein computerlesbares Speichermedium, auf welchem von einem System lesbare und ausführbare Programmabschnitte gespeichert sind, um alle Schritte des oben beschriebenen Verfahrens zum Bereitstellen eines bearbeiteten medizinischen Bildes und seine Aspekte auszuführen, wenn die Programmabschnitte von dem System ausgeführt werden.

Die oben beschriebenen Eigenschaften, Merkmale und Vorteile dieser Erfindung werden klarer und verständlicher im Zusammenhang mit folgenden Figuren und ihren Beschreibungen. Dabei sollen die Figuren und Beschreibungen die Erfindung und ihre Ausführungsformen in keiner Weise einschränken.

In verschiedenen Figuren sind gleiche Komponenten mit korrespondierenden Bezugszeichen versehen. Die Figuren sind in der Regel nicht maßstabsgetreu.

Es zeigen
Fig. 1 ein erstes Ausführungsbeispiel eines Verfahrens zum Bereitstellen eines bearbeiteten medizinischen Bildes,
Fig. 2 ein zweites Ausführungsbeispiel eines Verfahrens zum Bereitstellen eines bearbeiteten medizinischen Bildes,
Fig. 3 ein erstes Ausführungsbeispiel eines Verfahrensschrittes eines Bestimmens eines anatomischen Bereiches,
Fig. 4 ein zweites Ausführungsbeispiel eines Verfahrensschrittes eines Bestimmens eines anatomischen Bereiches,
Fig. 5 ein drittes Ausführungsbeispiel eines Verfahrens zum Bereitstellen eines bearbeiteten medizinischen Bildes,
Fig. 6 ein viertes Ausführungsbeispiel eines Verfahrens zum Bereitstellen eines bearbeiteten medizinischen Bildes,
Fig. 7 ein System zum Bereitstellen eines bearbeiteten medizinischen Bildes.

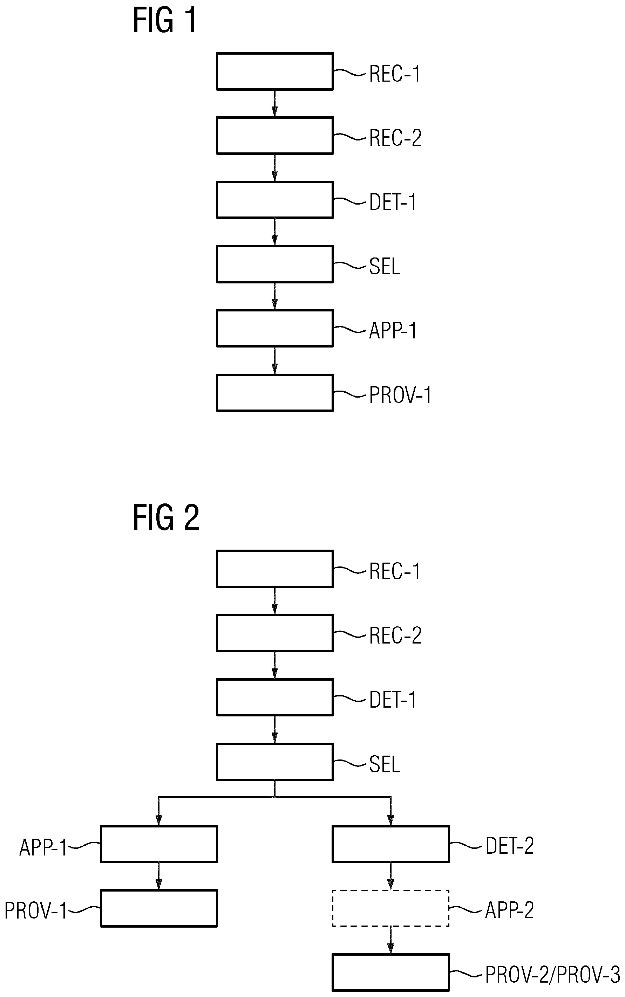

Figur 1 zeigt ein erstes Ausführungsbeispiel eines Verfahrens zum Bereitstellen eines bearbeiteten medizinischen Bildes.

In einem Verfahrensschritt eines Empfangens REC-1 eines medizinischen Bildes wird das medizinische Bild eines Patienten mittels einer Schnittstelle empfangen. Dabei bildet das medizinische Bild einen Untersuchungsbereich des Patienten ab. Der Untersuchungsbereich umfasst dabei wenigstens einen Teil des Patienten. Beispielsweise kann der Untersuchungsbereich der Thorax des Patienten oder der Schädel des Patienten oder das Abdomen des Patienten oder eine Gliedmaße des Patienten etc. sein. Das medizinische Bild kann dabei zwei- oder dreidimensional sein. Das medizinische Bild kann insbesondere ein zweidimensionales Röntgenbild, ein Ultraschallbild, ein Computer-Tomographie (Akronym: CT) Bild, ein Magnet-Resonanz-Tomographie (Akronym: MRT) Bild, ein Positronen-Emissions-Tomographie (Akronym: PET) Bild oder ein Einzel-Photonen-Emissions-Computer-Tomographie (engl. single photon emission computed tomography; Akronym: SPECT) Bild sein.

In einem Verfahrensschritt eines Empfangens REC-2 von Patienteninformationen werden mittels der Schnittstelle die Patienteninformationen zu dem Patienten empfangen. Die Patienteninformationen umfassen beispielsweise allgemeine Informationen zu dem Patienten. Beispielsweise können die Patienteninformationen Informationen über physische Merkmale des Patienten wie beispielsweise Alter und/oder Geschlecht des Patienten umfassen. Alternativ oder zusätzlich können die Patienteninformationen Informationen zu einer Krankheitsgeschichte des Patienten umfassen. Die Krankheitsgeschichte kann dabei beispielsweise durch Befunde, weitere medizinische Bilder, Diagnosen etc. beschrieben sein. Insbesondere können die Patienteninformationen eine Patientenakte des Patienten umfassen.

In einem weiteren Verfahrensschritt eines Bestimmens DET-1 eines anatomischen Bereiches wird mittels einer Recheneinheit der anatomische Bereich basierend auf den Patienteninformationen und dem medizinischen Bild bestimmt. Dabei umfasst der Untersuchungsbereich den anatomischen Bereich. Der anatomische Bereich ist somit auf dem medizinischen Bild abgebildet.

Der anatomische Bereich ist insbesondere zum Auswerten des medizinischen Bildes relevant. Beim Auswerten des medizinischen Bildes kann eine Diagnose oder ein Befund durch einen Nutzer, insbesondere einen Radiologen, erstellt werden. Der anatomische Bereich kann eine Auffälligkeit, beispielsweise eine Läsion oder eine Gruppe von Läsionen oder ein Tumor oder ein Fissur oder eine Verkalkung oder ein Gerinnsel etc., umfassen, welche zum Auswerten des medizinischen Bildes relevant ist.

Der anatomische Bereich kann dabei eine Körperstruktur umfassen, die die Auffälligkeit umfasst, beispielsweise einen Lungenlappen oder das Herz oder einen bestimmten Teil des Gehirns des Patienten. Alternativ kann der anatomische Bereich nur die Auffälligkeit umfassen.

Beim Bestimmen DET-1 des anatomischen Bereiches werden somit die Patienteninformationen und das medizinische Bild ausgewertet bzw. berücksichtig.

In einem weiteren Verfahrensschritt eines Auswählens SEL eines Werkzeuges wird ein Werkzeug basierend auf dem anatomischen Bereich ausgewählt. Das Werkzeug wird dabei aus einem Satz von verfügbaren Werkzeugen ausgewählt. Das Werkzeug ist zum Bearbeiten des medizinischen Bildes ausgebildet. Das Werkzeug ist dabei auf eine Analyse des anatomischen Bereiches in dem medizinischen Bild spezialisiert.

In einem weiteren Verfahrensschritt eines Anwendens APP-1 des ausgewählten Werkzeuges wird das Werkzeug mittels der Recheneinheit auf das medizinische Bild angewendet. Dabei wird ein bearbeitetes medizinisches Bild bestimmt.

In dem bearbeiteten medizinischen Bild kann beispielsweise der anatomische Bereich hervorgehoben bzw. markiert, insbesondere segmentiert sein. Wenn das medizinische Bild dreidimensional ist, kann das bearbeitet medizinische Bild diejenigen Schichten des dreidimensionalen medizinischen Bildes umfassen, die den anatomischen Bereich abbilden bzw. für eine Auswertung in Abhängigkeit des anatomischen Bereiches relevant sind. In Ausführungen der Erfindung kann in dem bearbeiteten medizinischen Bild beispielsweise ein Distanzmaß und/oder eine Abmessung eingezeichnet sein. Beispielsweise kann eine Abmessung, beispielsweise ein Durchmesser, einer Läsion in dem bearbeiteten medizinischen Bild eingezeichnet bzw. überlagert sein. Mit anderen Worten kann das bearbeitete medizinische Bild durch Anwenden des Werkzeuges annotiert sein.

In einem Verfahrensschritt eines Bereitstellens PROV-1 des bearbeiteten medizinischen Bildes wird das bearbeitete medizinische Bild mittels der Schnittstelle bereitgestellt. Dabei kann das bearbeitete medizinische Bild einer Anzeigeeinheit, insbesondere einem Bildschirm bzw. einem Monitor bereitgestellt werden. Dabei kann das bearbeitete medizinische Bild angezeigt werden. Insbesondere wird es dem Nutzer bzw. dem Radiologen angezeigt.

In Ausführungen der Erfindung umfassen die Patienteninformationen Einträge aus einer Patientenakte des Patienten und/oder wenigstens ein weiteres von dem medizinischen Bild verschiedenes medizinisches Bild des Patienten und/oder eine Befundungsaufgabe zu dem medizinischen Bild.

Die Einträge aus der Patientenakte können insbesondere Diagnosen und/oder Befunde und/oder allgemeine Informationen zu dem Patienten etc. umfassen. Mit anderen Worten können die Einträge aus der Patientenakte eine Krankheitsgeschichte des Patienten abbilden. Insbesondere können die Einträge aus der Patientenakte Informationen über an dem Patienten durchgeführte Untersuchungen umfassen.

Die Patientenakte kann dabei insbesondere eine elektronische Patientenakte sein. Die Patientenakte kann insbesondere in einem Krankenhausinformationssystem (engl. Hospital Information System, Akronym: HIS) hinterlegt bzw. gespeichert sein. Beim Empfangen der Patienteninformationen kann die Patientenakte aus dem HIS empfangen werden.

Das weitere medizinische Bild ist von dem medizinischen Bild verschieden. Das weitere medizinische Bild kann zu einem anderen Zeitpunkt als das medizinische Bild erfasst worden sein. Das weitere medizinische Bild kann wenigstens teilweise den gleichen Untersuchungsbereich wie das medizinische Bild abbilden. Alternativ kann das weitere medizinische Bild einen anderen Untersuchungsbereich abbilden. Das weitere medizinische Bild kann mit einer gleichen oder einer anderen Bildgebungsmodalität als das medizinische Bild erfasst worden sein. Die Bildgebungsmodalität wird dabei durch das Gerät bzw. System, mit welchem das medizinische Bild bzw. das weitere medizinische Bild erfasst worden ist, vorgegeben. Die Bildgebungsmodalität bestimmt insbesondere, welche physikalischen Eigenschaften des Untersuchungsbereiches in dem medizinischen Bild bzw. dem weiteren medizinischen Bild abgebildet sind.

Die Befundungsaufgabe gibt insbesondere an, zu welchem Zweck das medizinische Bild erfasst werden sollte. Insbesondere kann die Befundungsaufgabe angeben, wie, beispielsweise mit welcher Bildgebungsmodalität und/oder welcher Untersuchungsbereich und/oder welcher anatomische Bereich, das medizinische Bild erfasst werden sollte. Die Befundungsaufgabe kann angeben welche Diagnose mittels des medizinischen Bildes erstellt oder ausgeschlossen werden soll.

Figur 2 zeigt ein zweites Ausführungsbeispiel eines Verfahrens zum Bereitstellen eines bearbeiteten medizinischen Bildes.

Die Verfahrensschritte des Empfangens REC-1 eines medizinischen Bildes, des Empfangens REC-2 der Patienteninformationen, des Bestimmens DET-1 des anatomischen Bereiches, des Auswählens SEL des Werkzeuges, des Anwendens APP-1 des ausgewählten Werkzeuges auf das medizinische Bild und des Bereitstellens PROV-1 des bearbeiteten medizinischen Bildes sind gemäß der Beschreibung zu Figur 1 ausgebildet.

Das Verfahren umfasst einen weiteren Verfahrensschritt eines Bestimmens DET-2 derjenigen weiteren Bildes des Patienten aus den Patienteninformationen, die den anatomischen Bereich abbilden. Dabei umfassen die Patienteninformationen wenigstens ein weiteres medizinisches Bild des Patienten.

In dem Verfahrensschritt des Bestimmens DET-2 derjenigen weiteren medizinischen Bilder, die denselben anatomischen Bereich abbilden, werden mittels der Recheneinheit durch eine Bildanalyse diejenigen weiteren medizinischen Bilder in den Patienteninformationen bestimmt, die denselben anatomischen Bereich abbilden wie das medizinische Bild. Dabei können kein, ein oder mehrere weitere medizinische Bilder bestimmt werden.

In einem Verfahrensschritt eines Bereitstellens PROV-2 der bestimmten weiteren medizinischen Bilder, werden die derart bestimmten weiteren medizinischen Bilder aus den Patienteninformationen mittels der Schnittstelle bereitgestellt. Insbesondere werden dabei die bestimmten weiteren medizinischen Bilder einer Anzeigeeinheit bereitgestellt, die zum Anzeigen der weiteren medizinischen Bilder ausgebildet ist. Die weiteren medizinischen Bilder können parallel zu dem medizinischen Bild angezeigt werden. Auf diese Weise kann eine zeitliche Veränderung des anatomischen Bereiches dargestellt werden, wenn die bestimmten weiteren medizinischen Bilder und das medizinische Bild den anatomischen Bereich zu verschiedenen Zeitpunkten darstellen. Alternativ oder zusätzlich können die bestimmten weiteren medizinischen Bilder und das medizinische Bild verschiedene physikalische Eigenschaften des anatomischen Bereiches im Vergleich darstellen, wenn die bestimmten weiteren medizinischen Bilder und das medizinische Bild mit verschiedenen Bildgebungsmodalitäten erfasst wurden.

Gemäß einer Ausführung der Erfindung kann das Verfahren einen Verfahrensschritt eines Anwendens APP-2 des ausgewählten Werkzeuges auf die bestimmten weiteren medizinischen Bilder umfassen. Dabei wird das Werkzeug wie auch auf das medizinische Bild auf die bestimmten weiteren medizinischen Bilder angewendet. Dabei werden bearbeitete weitere medizinische Bilder bestimmt. Die bearbeiteten weiteren medizinischen Bildern sind analog zu dem bearbeiteten medizinischen Bild ausgebildet. Mit anderen Worten sind analoge Markierungen und/oder Segmentierungen und/oder Abmessungen und/oder Annotierungen in dem bearbeiteten weiteren medizinischen Bildern hinzugefügt wie auch in dem bearbeiteten medizinischen Bild.

Gemäß der Ausführung der Erfindung können dann in einem weiteren Verfahrensschritt eines Bereitstellens PROV-3 die bearbeiteten weiteren medizinischen Bilder bereitgestellt werden. Dabei werden die bearbeiteten weiteren medizinischen Bilder mittels der Schnittstelle insbesondere der Anzeigeeinheit zum Anzeigen der bearbeiteten weiteren medizinischen Bilder bereitgestellt. Dabei werden die bearbeiteten weiteren medizinischen Bilder gemeinsam mit dem bearbeiteten medizinischen Bild angezeigt. Insbesondere kann auf diese Weise eine zeitliche Veränderung des anatomischen Bereiches betont bzw. hervorgehoben bzw. vereinfacht dargestellt werden, wenn die bestimmten weiteren medizinischen Bilder und das medizinische Bild zu verschiedenen Zeitpunkten erfasst wurden. Alternativ oder zusätzlich können auf diese Weise verschiedenen physikalische Eigenschaften des anatomischen Bereiches betont bzw. hervorgehoben bzw. vereinfacht dargestellt werden, wenn die bestimmten weiteren medizinischen Bilder und das medizinische Bild mit verschiedenen Bildgebungsmodalitäten erfasst wurden.

Durch das Anzeigen eines zeitlichen Verlaufes ist es möglich vereinfacht eine zeitliche Entwicklung beispielsweise eine Krankheitsbefalls zu analysieren. Mit anderen Worten ist ein sogenanntes "Trending" vereinfacht und übersichtlich möglich.

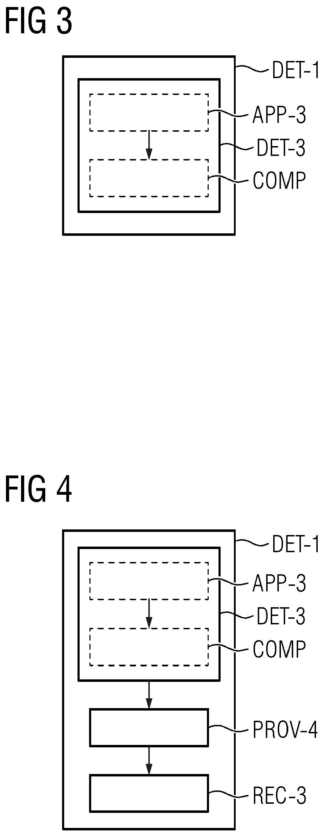

Figur 3 zeigt ein erstes Ausführungsbeispiel eines Verfahrensschrittes eines Bestimmens DET-1 eines anatomischen Bereiches.

Der Verfahrensschritt des Bestimmens DET-1 des anatomischen Bereiches ist grundlegend gemäß der Beschreibung zu Figur 1 ausgebildet.

Gemäß dem ersten Ausführungsbeispiel des Verfahrensschrittes des Bestimmens DET-1 des anatomischen Bereiches umfasst der Verfahrensschritt des Bestimmens DET-1 des anatomischen Bereiches einen Verfahrensschritt eines Bestimmens DET-3 eines Satzes von Observablen. Der Satz von Observablen wird dabei basierend auf den Patienteninformationen und dem medizinischen Bild bestimmt. Das Bestimmen DET-1 des anatomischen Bereiches basiert dann auf dem Satz von Observablen. Mit anderen Worten wird der anatomische Bereich in Abhängigkeit des Satzes von Observablen bestimmt.

Der Satz von Observablen umfasst wenigstens eine Observable. Der Satz von Observablen umfasst Eigenschaften, die aus den Patienteninformationen und dem medizinischen Bild extrahiert werden können. Insbesondere kann der Satz von Observablen eine Sammlung der wichtigsten Eigenschaften aus den Patienteninformationen und dem medizinischen Bild umfassen.

In Ausführungen der Erfindung kann eine Observable aus dem Satz von Observablen eine systeminterne Observable oder ein Eintrag in einem dem medizinischen Bild zugeordneten Satz von Eigenschaften oder ein Körperteil gemäß einem Befund gemäß der Patienteninformationen oder ein auffälliger Laborwert gemäß der Patienteninformationen oder ein Körperbereich, von dem gemäß den Patienteninformationen eine Gewebeprobe genommen wurde, oder eine Verdachtsdiagnose oder ein Befund gemäß der Patienteninformationen oder ein Stichwort aus einer Befundungsaufgabe für das medizinische Bild sein.

Eine systeminterne Observable kann dabei auf einer vorbestimmten, insbesondere standardisierten, anatomischen Ontologie basieren. Ferner können ein oder mehrere systeminterne Observablen entsprechend der vorbestimmten anatomischen Ontologie hierarchisch miteinander verknüpft sein. Die vorbestimmten anatomische Ontologie kann auf der "SNOMED Clinical Terms" Terminologie bzw. Ontologie und/oder der "RadLex" Terminologie bzw. Ontologie basieren. Die Verwendung dieser an sich bekannten standardisierten Terminologien zur Definition bzw. zum Aufbau der Observablen stellt die Kompatibilität mit den klinischen Abläufen sicher, verbessert den Austausch von Informationen und erleichtert die Zuordnung des anatomischen Bereichs.

Der Satz von Eigenschaften kann insbesondere ein DICOM Header des medizinischen Bildes sein. Der DICOM Header kann dabei eine Mehrzahl von Einträgen bzw. einen Satz von Einträgen umfassen. Jeder Eintrag kann dabei durch einen DICOM Tag und einen DICOM Wert definiert sein. Der DICOM Tag definiert dabei die Eigenschaft, die durch den DICOM Wert präzisiert bzw. ausgeführt wird. Beispielsweise kann ein DICOM Eintrag einen DICOM Tag "Körperregion" und einen DICOM Wert "Thorax" umfassen. Auf diese Weise wird angegeben, dass in dem medizinischen Bild die Körperregion "Thorax" abgebildet ist. Ein für die Auswertung des medizinischen Bildes relevanter Eintrag kann eine Observable in dem Satz von Observablen bilden. Insbesondere kann der DICOM Wert des Eintrags die Observable bilden.

Die Observable kann ein Stichwort sein, welches eine der oben genannten Eigenschaften bzw. Angaben beschreibt. Dabei kann das Stichwort in Form einer systeminternen Observablen angegeben sein. Mit anderen Worten kann beispielsweise der Körperteil oder der Befund oder die Diagnose in Form einer systeminternen Observablen angegeben sein. Für eine Diagnose oder einen Befund kann die systeminterne Observable als ICD 10 Code angegeben sein.

Gemäß einer Ausführung der Erfindung kann für eine Mehrzahl von anatomischen Bereichen Sätze von typischen Observablen vorgegeben sein. Dabei ist für jeden anatomischen Bereich der Mehrzahl von anatomischen Bereichen ein Satz von typischen Observablen vorgegeben. Beim Bestimmen DET-1 des anatomischen Bereiches können die Sätze von typischen Observablen mit dem bestimmten Satz von Observablen verglichen werden. Mittels Mustererkennung kann dabei der Satz von typischen Observablen bestimmt werden, der am besten mit dem bestimmten Satz von Observablen übereinstimmt bzw. die größte Übereinstimmung aufweist. Der anatomische Bereich, dem dieser Satz von typischen Observablen zugeordnet ist, ist dann der bestimmte anatomische Bereich.

In einer Ausführung der Erfindung umfasst der Verfahrensschritt des Bestimmens DET-3 des Satzes von Observablen einen Verfahrensschritt eines Anwendens APP-3 einer linguistischen Analysefunktion auf wenigstens einen Eintrag aus der Patientenakte und/oder eine Befundungsaufgabe. Dabei umfassen die Patienteninformationen den wenigstens einen Eintrag aus der Patientenakte und/oder die Befundungsaufgabe. Dabei wird wenigstens ein Schlagwort bestimmt. Der Verfahrensschritt des Bestimmens DET-3 des Satzes von Observablen umfasst dann außerdem einen Verfahrensschritt eines Abgleichens COMP des wenigstens einen Schlagwortes mit dem Satz von Eigenschaften. Bei einem positiven Abgleich wird das Schlagwort in den Satz von Observablen als Observable aufgenommen.

De linguistische Analysefunktion ist dazu ausgebildet, den Eintrag aus der Patientenakte und/oder die Befundungsaufgabe linguistisch zu analysieren. Das Schlagwort kann dann eine Zusammenfassung bzw. die wichtigste Eigenschaft bzw. Information aus dem Eintrag der Patientenakte und/oder der Befundungsaufgabe sein. Das Schlagwort kann in Form eines Wortes oder in Form einer oben beschriebenen Ontologie ausgebildet sein.

Bei dem Abgleichen COM des Schlagwortes mit dem Satz von Eigenschaften wird überprüft, ob der Satz von Eigenschaften das Schlagwort selbst oder ein Synonym des Schlagwortes umfasst. Der Satz von Eigenschaften kann dabei wie oben beschrieben ein DICOM Header sein. Der Abgleich kann dabei insbesondere auf den DICOM Werten basieren. Bei einem positiven Abgleich umfasst der Satz von Eigenschaften das Schlagwort oder ein Synonym des Schlagwortes. Dann wird das Schlagwort oder das Synonym des Schlagwortes oder eine verallgemeinerte Form des Schlagwortes in den Satz von Observablen aufgenommen. Mit anderen Worten bildet dann das Schlagwort bzw. das Synonym des Schlagwortes bzw. die Verallgemeinerung des Schlagwortes eine Observable in dem Satz von Eigenschaften aus. Die Verallgemeinerung des Schlagwortes kann insbesondere eine Umwandlung des Schlagwortes in eine systeminterne Observable umfassen.

Figur 4 zeigt ein zweites Ausführungsbeispiel eines Verfahrensschrittes eines Bestimmens eines anatomischen Bereiches.

Der Verfahrensschritt des Bestimmens DET-1 des anatomischen Bereiches ist grundlegend wie in den Beschreibungen zu den Figuren 1 und 3 ausgebildet. Der Verfahrensschritt des Bestimmens DET-3 des Satzes von Observablen ist gemäß der Beschreibung zu Figur 3 ausgebildet.

Der Verfahrensschritt des Bestimmens DET-1 des anatomischen Bereiches umfasst gemäß dem im Folgenden beschriebenen Ausführungsbeispiel einen Verfahrensschritt eines Bereitstellens PROV-4 des Satzes von Observablen und einen Verfahrensschritt eines Empfangens REC-3 einer Nutzereingabe bezüglich des Satzes von Observablen. Dabei wird die Nutzereingabe beim Bestimmen DET-1 des anatomischen Bereiches berücksichtigt.

In dem Verfahrensschritt des Bereitstellens PROV-4 des Satzes von Observablen wird der Satz von Observablen insbesondere mittels der Schnittstelle bereitgestellt. Dabei kann der Satz von Observablen insbesondere der Anzeigeeinheit zum Anzeigen der Observablen bereitgestellt werden. Der Satz von Observablen wird dabei dem Nutzer, insbesondere dem Radiologen bereitgestellt.

Die Nutzereingabe wird in dem Verfahrensschritt des Empfangens REC-3 der Nutzereingabe mittels der Schnittstelle empfangen. Die Nutzereingabe wird dabei von dem Nutzer, insbesondere dem Radiologen, bereitgestellt. Die Nutzereingabe kann durch den Nutzer über eine Eingabeeinheit, beispielsweise einen berührungsempfindlichen Bildschirm (eng. Touchscreen) oder ein Tastfeld (engl. Touchpad) oder eine Mauseingabe etc., bereitgestellt werden. Die Nutzereingabe bezieht sich dabei auf den Satz von Observablen. Die Nutzereingabe kann dabei dazu ausgebildet sein, den Satz von Observablen zu bestätigen oder zu modifizieren. Der anatomische Bereich wird dann basierend auf dem bestätigten oder dem modifizierten Satz von Observablen bestimmt.

In einer alternativen Ausführung der Erfindung kann zunächst der anatomische Bereich basierend auf dem bestimmten Satz von Observablen wie oben beschrieben bestimmt werden. In dem Verfahrensschritt des Bereitstellens PROV-4 des Satzes von Observablen kann dann zusätzlich der bestimmte anatomische Bereich bereitgestellt werden. Die Nutzereingabe kann dann zusätzlich den bereits bestimmten anatomischen Bereich berücksichtigen und den Satz von Observablen entsprechend bestätigen oder modifizieren. Der anatomische Bereich kann dann basierend auf dem bestätigten oder modifizierten Satz von Observablen erneut bestimmt werden.

In Ausführungen der Erfindung umfasst die Nutzereingabe bezüglich des Satzes von Observablen ein Priorisieren und/oder ein Löschen und/oder ein Auswählen der Observablen.

Beim Priorisieren werden durch die Nutzereingabe die Observablen des Satzes von Observablen priorisiert. Durch das Priorisieren kann angegeben werden, wie stark die jeweiligen Observablen beim Bestimmen DET-1 des anatomischen Bereiches berücksichtigt werden sollen. Wenn der anatomische Bereich beispielsweise wie gemäß der Beschreibung zu Figur 3 beschrieben mittels Mustererkennung bestimmt wird, kann mittels der Priorisierung vorgegeben werden, welche Observablen bei dem Abgleich des Satzes von Observablen mit den Sätzen von typischen Observablen besonders berücksichtigt werden sollen.

Beim Löschen können durch die Nutzereingabe ein oder mehrere Observablen aus dem Satz von Observablen gelöscht werden.

Beim Auswählen können ein oder mehrere Observablen aus dem Satz von Observablen ausgewählt werden. Dabei kann der Satz von Observablen derart modifiziert werden, dass er nur noch die ausgewählten Observablen umfasst und alle anderen Observablen aus dem Satz von Observablen gelöscht bzw. entfernt werden. Alternativ oder zusätzlich kann das Auswählen einer Observablen eine Observable zu dem Satz von Observablen hinzufügen. Mit anderen Worten kann auf diese Weise eine Observable, die bisher noch nicht von dem Satz von Observablen umfasst wer, zu dem Satz von Observablen hinzugefügt werden.

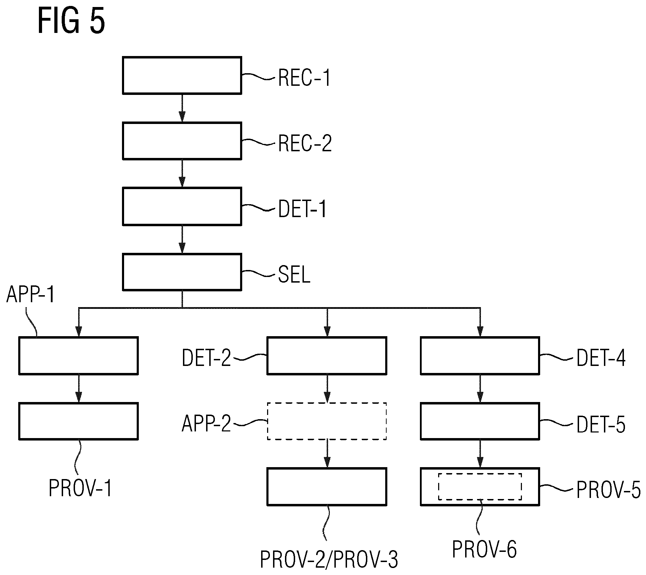

Figur 5 zeigt ein drittes Ausführungsbeispiel eines Verfahrens zum Bereitstellen eines bearbeiteten medizinischen Bildes.

Die bereits in den Beschreibungen zu den Figuren 1 oder 2 beschriebenen Verfahrensschritte sind gemäß der Beschreibung ausgebildet. Der Verfahrensschritt des Bestimmens DET-1 des anatomischen Bereiches kann dabei gemäß einem der Ausführungsbeispiele aus den Figuren 3 oder 4 ausgebildet sein.

Alternativ können die im Folgenden beschriebenen zusätzlichen Verfahrensschritte auch mit dem ersten Ausführungsbeispiel gemäß Figur 1 ausgebildet sein.

In einem weiteren Verfahrensschritt eines Bestimmens DET-4 eines Suchprofils wird mittels der Recheneinheit ein Suchprofil für ähnliche Patienten basierend auf den Patienteninformationen und/oder dem medizinischen Bild bestimmt. Mit anderen Worten wird ein Suchprofil bestimmt, mit welchem nach einem oder mehreren dem Patienten ähnliche Patienten gesucht werden kann.

Ein ähnlicher Patient kann ähnliche Eigenschaften und/oder Diagnosen und/oder Befunde und/oder Untersuchungen wie der Patient aufweisen.

Das Suchprofil definiert dabei die Eigenschaften und/oder Diagnosen und/oder Befunde und/oder Untersuchungen etc. die der ähnliche Patient aufweisen sollte.

Dafür kann in Ausführungen der Erfindung wie in den vorstehenden Figuren beschrieben ein Satz von Observablen bestimmt werden. Basierend auf diesem Satz von Observablen kann dann das Suchprofil nach den ähnlichen Patienten erstellt werden.

Alternativ kann das Suchprofil durch Anwenden einer trainierten Funktion auf die Patienteninformationen und das medizinische Bild bestimmt werden. Die trainierte Funktion kann dabei mittels föderalem Lernen (engl. federated learning) trainiert worden sein.

In einer alternativen Ausführungsform kann das Suchprofil alternativ oder zusätzlich basierend auf dem bestimmten anatomischen Bereich bestimmt werden. Mit anderen Worten berücksichtigt das Suchprofil den bestimmten anatomischen Bereich. Insbesondere kann dann mit dem Suchprofil nach einem oder mehreren ähnlichen Patienten gesucht werden, bei welchen ebenfalls wenigstens eine Untersuchung desselben anatomischen Bereiches durchgeführt wurde und/oder ein medizinisches Bild desselben anatomischen Bereiches erfasst wurde.

In einem weiteren Verfahrensschritt eines Bestimmens DET-5 von wenigstens einem ähnlichen Patienten wird mittels der Recheneinheit wenigstens ein ähnlicher Patient aus mehreren Referenz-Patienten bestimmt. Der wenigstens eine ähnliche Patient wird dabei durch Anwenden des Suchprofils in einem medizinischen Informationssystem, in welchem die Referenz-Patienten angelegt sind, bestimmt.

Das medizinische Informationssystem kann dabei insbesondere ein PACS oder ein LIS oder ein HIS oder ein RIS sein. Mit anderen Worten ist das medizinische Informationssystem eine Datenbank.

"Angelegt" bedeutet, dass Informationen bezüglich der Referenz-Patienten in dem medizinischen Informationssystem hinterlegt bzw. gespeichert sind. Dabei ist jedem Referenz-Patienten ein Patientenidentifikator zugeordnet, der einen Zugriff auf die Informationen bezüglich des Referenz-Patienten ermöglicht.

Durch Anwenden des Suchprofils werden diejenigen Referenz-Patienten bestimmt, die die Anforderungen des Such-Profils erfüllen. Insbesondere werden somit diejenigen Referenz-Patienten bestimmt, die die in dem Suchprofil vorgegebenen Eigenschaften und/oder Diagnosen und/oder Befunde und/oder Untersuchungen erfüllen. Dabei werden kein, ein oder mehrere Referenz-Patienten bestimmt. Diese Referenz-Patienten werden als ähnliche Patienten zu dem Patienten bezeichnet.

In einem weiteren Verfahrensschritt eines Bereitstellens PROV-5 einer mit dem wenigstens einen ähnlichen Patienten in Zusammenhang stehende Information wird die Information zu dem ähnlichen Patient mittels der Schnittstelle bereitgestellt. Die mit dem ähnlichen Patienten in Zusammenhang stehende Information ist dabei insbesondere eine Patienteninformation oder ein medizinisches Bild des ähnlichen Patienten. Die Patienteninformation und das ähnliche Bild sind dabei wie gemäß der Beschreibung zu Figur 1 bezüglich dem Patienten beschrieben ausgebildet. Die mit dem ähnlichen Patienten in Zusammenhang stehende Information kann insbesondere der Anzeigeeinheit zum Anzeigen bereitgestellt werden.

Gemäß einer Ausführung der Erfindung umfasst die mit dem ähnlichen Patienten in Zusammenhang stehende Information eine Diagnose des ähnlichen Patienten. Der Verfahrensschritt des Bereitstellens PROV-5 der mit dem ähnlichen Patienten in Zusammenhang stehenden Information kann dann einen Verfahrensschritt eines Bereitstellens PROV-6 der Diagnose des ähnlichen Patienten umfassen. Auf diese Weise kann eine Information darüber bereitgestellt werden, welche Diagnose für einen ähnlichen Patienten in einer möglicherweise ähnlichen Situation erstellt wurde. Diese Information kann gemeinsam mit dem bearbeiteten medizinischen Bild bereitgestellt, insbesondere angezeigt werden.

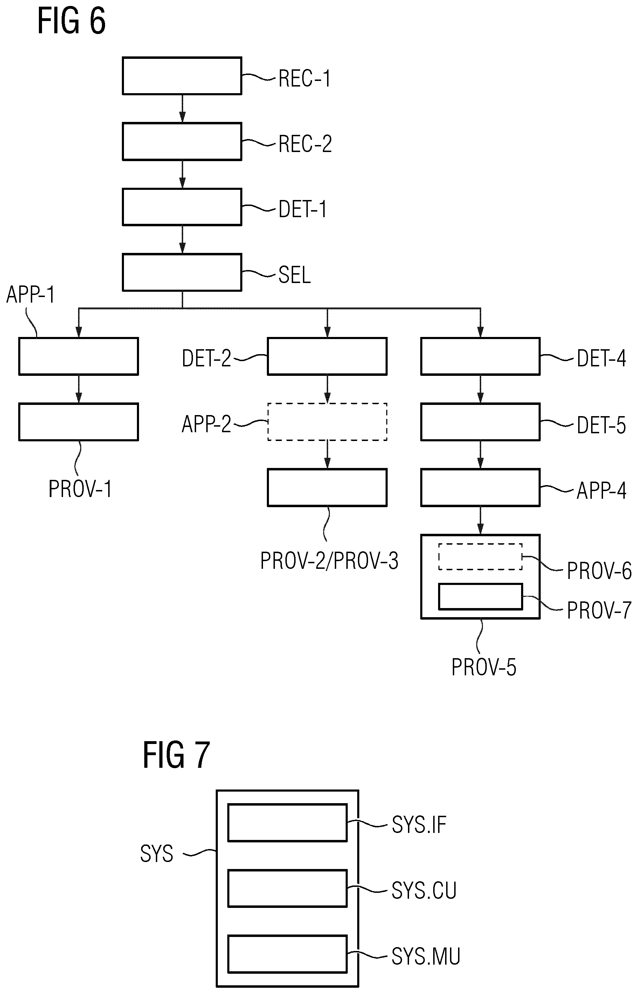

Figur 6 zeigt ein viertes Ausführungsbeispiel eines Verfahrens zum Bereitstellen eines bearbeiteten medizinischen Bildes.

Die bereits in den Beschreibungen zu den Figuren 1, 2 oder 5 beschriebenen Verfahrensschritte sind gemäß der Beschreibung ausgebildet. Der Verfahrensschritt des Bestimmens DET-1 des anatomischen Bereiches kann dabei gemäß einem der Ausführungsbeispiele aus den Figuren 3 oder 4 ausgebildet sein.

Alternativ können die im Folgenden beschriebenen zusätzlichen Verfahrensschritte auch mit dem ersten Ausführungsbeispiel gemäß Figur 1 ausgebildet sein.

Die mit dem ähnlichen Patienten in Zusammenhang stehende Information umfasst wenigstens ein medizinisches Bild des ähnlichen Patienten. Dabei ist vorteilhafterweise die Befundungsaufgabe bezüglich des wenigstens einen medizinischen Bildes des ähnlichen Patienten gleich oder ähnlich zu der Befundungsaufgabe des medizinischen Bildes. Insbesondere bildet das wenigstens eine medizinische Bild des ähnlichen Patienten vorteilhafterweise den bestimmten anatomischen Bereich des ähnlichen Patienten ab.

In einem Verfahrensschritt eines Anwendens APP-4 des ausgewählten Werkzeuges auf das medizinische Bild des ähnlichen Patienten wird das Werkzeug mittels der Recheneinheit auf das medizinische Bild des ähnlichen Patienten angewendet. Dabei wird ein bearbeitetes medizinisches Bild des ähnlichen Patienten bestimmt. Das bearbeitete medizinische Bild des ähnlichen Patienten ist dabei analog zu dem bearbeiteten medizinischen Bild des Patienten bearbeitet. Mit anderen Worten umfassen beide Bilder die gleichen Markierungen und/oder Annotationen und/oder Abmessungen etc.

In einem Verfahrensschritt eines Bereitstellens PROV-7 des bearbeiteten medizinischen Bildes des ähnlichen Patienten wird das derart bearbeitete medizinische Bild des ähnlichen Patienten mittels der Schnittstelle bereitgestellt. Insbesondere wird das bearbeitete medizinische Bild des ähnlichen Patienten mit der Anzeigeeinheit angezeigt. Vorteilhafterweise wird das bearbeitete medizinische Bild des ähnlichen Patienten gemeinsam mit dem bearbeiteten medizinischen Bild des Patienten angezeigt.

Figur 7 zeigt ein System SYS zum Bereitstellen eines bearbeiteten medizinischen Bildes.

Das dargestellte System SYS zum Bereitstellen eines bearbeiteten medizinischen Bildes ist dazu ausgebildet ein erfindungsgemäßes Verfahren zum Bereitstellen eines bearbeiteten medizinischen Bildes auszuführen. Das System SYS umfasst eine Schnittstelle SYS.IF, eine Recheneinheit SYS.CU und eine Speichereinheit SYS.MU.

Das System SYS kann insbesondere ein Computer, ein Mikrocontroller oder ein integrierter Schaltkreis (integrated circuit, IC) sein. Alternativ kann das System SYS ein reales oder virtuelles Computer-Netzwerk sein (eine technische Bezeichnung für ein reales Computer-Netzwerk ist "Cluster", eine technische Bezeichnung für ein virtuelles Computer-Netzwerk ist "Cloud"). Das System SYS kann als virtuelles System ausgebildet sein, welches auf einem Computer oder einem realen Computer-Netzwerk oder einem virtuellen Computer-Netzwerk ausgeführt wird (eine technische Bezeichnung ist "Virtualization") .

Die Schnittstelle SYS.IF kann eine Hardware- oder Software-Schnittstelle sein (beispielsweise ein PCI bus, USB oder Firewire). Die Recheneinheit SYS.CU kann Hardware und/oder Software Bestandteile umfassen, beispielsweise einen Mikroprozessor oder einen sogenannten FPGA (Field Programmable Gate Way). Die Speichereinheit SYS.MU kann als nicht permanent arbeitender Arbeitsspeicher (Random Access Memory, RAM) oder als permanenter Massenspeicher (Festplatte, USB-Stick, SD-Karte, Solid State Disk (SSD)) ausgebildet sein.

Die Schnittstelle SYS.IF kann insbesondere eine Mehrzahl an Sub-Schnittstellen umfassen, die unterschiedliche Verfahrensschritte des jeweiligen erfindungsgemäßen Verfahrens ausführen. Mit anderen Worten kann die Schnittstelle SYS.IF als eine Mehrzahl an Schnittstellen SYS.IF ausgebildet sein. Die Recheneinheit SYS.CU kann insbesondere eine Mehrzahl an Sub-Recheneinheiten umfassen, die unterschiedliche Verfahrensschritte des jeweiligen erfindungsgemäßen Verfahrens ausführen. Mit anderen Worten kann die Recheneinheit SYS.CU als eine Mehrzahl an Recheneinheiten SYS.CU ausgebildet sein.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel be-schriebene Vorteile der Erfindung treffen ohne explizite Nennung, wo übertragbar, auch auf andere Ausführungsbeispiele zu.

## Patentansprüche

1. Computerimplementiertes Verfahren zum Bereitstellen eines bearbeiteten medizinischen Bildes,
umfassend folgende Verfahrensschritte:
- Empfangen (REC-1) eines medizinischen Bildes,
wobei das medizinische Bild einen Untersuchungsbereich eines Patienten abbildet und wobei das medizinische Bild ein zweidimensionales Röntgenbild, ein Ultraschallbild, ein Computer-Tomographie (CT) Bild, ein Magnet-Resonanz-Tomographie (MRT) Bild, ein Positronen-Emissions-Tomographie (PET) Bild oder ein Einzel-Photonen-Emissions-Computer-Tomographie (SPECT) Bild ist,
- Empfangen (REC-2) von Patienteninformationen des Patienten,
- Bestimmen (DET-1) eines anatomischen Bereiches basierend auf dem medizinischen Bild und den Patienteninformationen, wobei der Untersuchungsbereich den anatomischen Bereich umfasst und wobei der anatomische Bereich sich auf eine Organstruktur oder ein Körperteil bezieht, der einen auffälligen Bereich oder eine auffällige Struktur umfasst,
- Auswählen (SEL) eines Werkzeuges basierend auf dem anatomischen Bereich,
wobei das Werkzeug zum Bearbeiten des in dem medizinischen Bild abgebildeten anatomischen Bereichs ausgebildet ist,
- Anwenden (APP-1) des ausgewählten Werkzeuges auf das medizinische Bild,
wobei ein bearbeitetes medizinisches Bild bestimmt wird,
- Bereitstellen (PROV-1) des bearbeiteten medizinischen Bildes.

2. Verfahren nach Anspruch 1,
wobei die Patienteninformationen Einträge aus einer Patientenakte des Patienten und/oder wenigstens ein weiteres von dem medizinischen Bild verschiedenes medizinisches Bild des Patienten und/oder eine Befundungsaufgabe zu dem medizinischen Bild umfassen.

3. Verfahren nach Anspruch 2,
außerdem umfassend folgende Verfahrensschritte:
- Bestimmen (DET-2) derjenigen weiteren medizinischen Bilder des Patienten aus den Patienteninformationen, die den anatomischen Bereich abbilden,
- Bereitstellen (PROV-2) der bestimmten weiteren medizinischen Bilder.

4. Verfahren nach Anspruch 3,
außerdem umfassend folgenden Verfahrensschritt:
- Anwenden (APP-2) des ausgewählten Werkzeuges auf die bestimmten weiteren medizinischen Bilder, die den anatomischen Bereich abbilden,
- Bereitstellen (PROV-3) der bearbeiteten weiteren medizinischen Bilder.

5. Verfahren nach einem der vorhergehenden Ansprüche,
wobei der Verfahrensschritt des Bestimmens (DET-1) des anatomischen Bereiches folgenden Verfahrensschritt umfasst:
- Bestimmen (DET-3) eines Satzes von Observablen basierend auf dem medizinischen Bild und den Patienteninformationen, wobei das Bestimmen (DET-1) des anatomischen Bereiches auf dem Satz von Observablen basiert.

6. Verfahren nach Anspruch 5,
wobei eine Observable aus dem Satz an Observablen eine systeminterne Observable oder ein Eintrag in einem dem medizinischen Bild zugeordneten Satz von Eigenschaften oder ein Körperteil gemäß einem Befund gemäß der Patienteninformationen oder ein auffälliger Laborwert gemäß der Patienteninformationen oder ein Körperbereich, von dem gemäß der Patienteninformationen eine Gewebeprobe genommen wurde, oder eine Verdachtsdiagnose oder ein Befund gemäß der Patienteninformationen oder ein Stichwort aus einer Befundungsaufgabe für das medizinische Bild, ist.

7. Verfahren nach einem der Ansprüche 5 oder 6,
wobei in dem Verfahrensschritt des Bestimmens (DET-1) des anatomischen Bereiches der anatomische Bereich mittels Mustererkennung aus einer Mehrzahl von anatomischen Bereichen bestimmt wird,
wobei jedem der anatomischen Bereiche der Mehrzahl von anatomischen Bereichen ein Satz von typischen Observablen zugeordnet ist,
wobei derjenige anatomische Bereich mittels Mustererkennung aus der Mehrzahl von anatomischen Bereichen bestimmt wird, für den der zugeordnete Satz von typischen Observablen am besten mit dem basierend auf dem medizinischen Bild und den Patienteninformationen bestimmten Satz von Observablen übereinstimmt.

8. Verfahren nach einem der Ansprüche 5 bis 7,
wobei die Patienteninformationen wenigstens einen Eintrag aus einer Patientenakte und/oder eine Befundungsaufgabe umfassen, wobei dem medizinischen Bild ein Satz von Eigenschaften zugeordnet ist,
wobei der Verfahrensschritt des Bestimmens (DET-3) des Satzes von Observablen folgenden Verfahrensschritt umfasst:
- Anwenden (APP-3) einer linguistischen Analysefunktion auf den wenigstens einen Eintrag aus der Patientenakte und/oder die Befundungsaufgabe,
wobei wenigstens ein Schlagwort bestimmt wird,
- Abgleichen (COMP) des wenigstens einen Schlagwortes mit dem Satz von Eigenschaften,
wobei bei einem positiven Abgleich das Schlagwort in den Satz von Observablen aufgenommen wird.

9. Verfahren nach einem der Ansprüche 5 bis 8,
außerdem umfassend folgende Verfahrensschritte:
- Bereitstellen (PROV-4) des Satzes von Observablen,
- Empfangen (REC-3) einer Nutzereingabe bezüglich des Satzes von Observablen,
wobei die Nutzereingabe beim Bestimmen des anatomischen Bereiches berücksichtigt wird.

10. Verfahren nach Anspruch 9,
wobei die Nutzereingabe bezüglich des Satzes von Observablen ein Priorisieren und/oder ein Löschen und/oder ein Auswählen der Observablen umfasst.

11. Verfahren nach einem der vorhergehenden Ansprüche, außerdem umfassend folgende Verfahrensschritte:
- Bestimmen (DET-4) eines Suchprofils für ähnliche Patienten basierend auf den Patienteninformationen und/oder dem medizinischen Bild,
- Bestimmen (DET-5) von wenigstens einem ähnlichen Patienten aus mehreren Referenz-Patienten durch Anwenden des Suchprofils in einem medizinischen Informationssystem, in welchem die verschiedenen Referenz-Patienten angelegt sind,
- Bereitstellen (PROV-5) einer mit dem wenigstens einen ähnlichen Patienten im Zusammenhang stehenden Information.

12. Verfahren nach Anspruch 11,
wobei die mit dem wenigstens einen ähnlichen Patienten in Zusammenhang stehende Information eine Diagnose des ähnlichen Patienten umfasst,
wobei der Verfahrensschritt des Bereitstellens (PROV-5) einer mit dem ähnlichen Patienten in Zusammenhang stehenden Information folgenden Verfahrensschritt umfasst:
- Bereitstellen (PROV-6) der Diagnose des ähnlichen Patienten.

13. Verfahren nach einem der Ansprüche 11 oder 12,
wobei die mit dem wenigstens einen ähnlichen Patienten in Zusammenhang stehende Information ein medizinisches Bild des ähnlichen Patienten umfasst,
wobei das Verfahren außerdem folgende Verfahrensschritte umfasst:
- Anwenden (APP-4) des ausgewählten Werkzeuges auf das medizinische Bild des ähnlichen Patienten,
wobei ein bearbeitetes medizinisches Bild des ähnlichen Patienten bestimmt wird,
- Bereitstellen (PROV-7) des bearbeiteten medizinischen Bildes des ähnlichen Patienten.

14. System (SYS) zum Bereitstellen eines bearbeiteten medizinischen Bildes,
umfassend eine Schnittstelle (SYS.IF) und eine Recheneinheit (SYS.CU),
wobei die Schnittstelle (SYS.IF) und die Recheneinheit (SYS.CU) zum Ausführen der folgenden Verfahrensschritte ausgebildet sind:
- Empfangen (REC-1) eines medizinischen Bildes,
wobei das medizinische Bild einen Untersuchungsbereich eines Patienten abbildet und wobei das medizinische Bild ein zweidimensionales Röntgenbild, ein Ultraschallbild, ein Computer-Tomographie (CT) Bild, ein Magnet-Resonanz-Tomographie (MRT) Bild, ein Positronen-Emissions-Tomographie (PET) Bild oder ein Einzel-Photonen-Emissions-Computer-Tomographie (SPECT) Bild ist,
- Empfangen (REC-2) von Patienteninformationen des Patienten,
- Bestimmen (DET-1) eines anatomischen Bereiches basierend auf dem medizinischen Bild und den Patienteninformationen, wobei der Untersuchungsbereich den anatomischen Bereich umfasst und wobei der anatomische Bereich sich auf eine Organstruktur oder ein Körperteil bezieht, der einen auffälligen Bereich oder eine auffällige Struktur umfasst,
- Auswählen (SEL) eines Werkzeuges basierend auf dem anatomischen Bereich,
wobei das Werkzeug zum Bearbeiten des in dem medizinischen Bild abgebildeten anatomischen Bereichs ausgebildet ist,
- Anwenden (APP-1) des ausgewählten Werkzeuges auf das medizinische Bild,
wobei ein bearbeitetes medizinisches Bild bestimmt wird,
- Bereitstellen (PROV-1) des bearbeiteten medizinischen Bildes.

15. Computerprogrammprodukt mit einem Computerprogramm, welches direkt in einen Speicher (SYS.CU) eines Systems (SYS) ladbar ist, mit Programmabschnitten, um alle Schritte des Verfahrens nach einem der Ansprüche 1 bis 13 auszuführen, wenn die Programmabschnitte von dem System (SYS) ausgeführt werden

16. Computerlesbares Speichermedium, auf welchem von einem System (SYS) lesbare und ausführbare Programmabschnitte gespeichert sind, um alle Schritte des Verfahrens nach einem der Ansprüche 1 bis 13 auszuführen, wenn die Programmabschnitte von dem System (SYS) ausgeführt werden.

## Claims

1. Computer-implemented method for providing an edited medical image,
comprising the following method steps:
- receiving (REC-1) a medical image,
wherein the medical image depicts an examination region of a patient and wherein the medical image is a two-dimensional X-ray image, an ultrasound image, a computed tomography (CT) image, a magnetic resonance tomography (MRT) image, a positron emission tomography (PET) image, or a single photon emission computed tomography (SPECT) image,
- receiving (REC-2) patient information of the patient;
- determining (DET-1) an anatomical region on the basis of the medical image and the patient information,
wherein the examination region comprises said anatomical region and wherein the anatomical region relates to an organ structure, or to a body part that comprises an abnormal region or an abnormal structure,
- selecting (SEL) a tool on the basis of the anatomical region,
wherein the tool is designed to edit anatomical region depicted in the medical image;
- applying (APP-1) the selected tool to the medical image,
wherein an edited medical image is determined,
- providing (PROV-1) the edited medical image.

2. Method according to claim 1,
wherein the patient information comprises entries from a patient file of the patient, and/or at least one further medical image of the patient, which differs from the medical image, and/or a diagnostic task associated with the medical image.

3. Method according to claim 2,
also comprising the following method steps:
- determining (DET-2) from the patient information those further medical images of the patient that depict the anatomical region;
- providing (PROV-2) the determined further medical images.

4. Method according to claim 3,
also comprising the following method step:
- applying (APP-2) the selected tool to the determined further medical images that depict the anatomical region;
- providing (PROV-3) the edited further medical images.

5. Method according to one of the preceding claims,
wherein the method step of determining (DET-1) the anatomical region comprises the following method step:
- determining (DET-3) a set of observables on the basis of the medical image and the patient information,
wherein the determining (DET-1) of the anatomical region is based on the set of observables.

6. Method according to claim 5,
wherein an observable from the set of observables is a system-internal observable, or an entry in a set of attributes that is associated with the medical image, or a body part according to a medical finding according to the patient information, or an abnormal laboratory value according to the patient information, or a body region from which, according to the patient information, a tissue sample was taken, or a suspected diagnosis or medical finding according to the patient information, or a keyword from a diagnostic task for the medical image.

7. Method according to one of claims 5 or 6,
wherein in the method step of determining (DET-1) the anatomical region, pattern recognition is used to determine the anatomical region from a plurality of anatomical regions, wherein each of the anatomical regions of the plurality of anatomical regions is associated with a set of typical observables,
wherein pattern recognition is used to determine from the plurality of anatomical regions, that anatomical region for which the associated set of typical observables best matches the set of observables determined on the basis of the medical image and the patient information.

8. Method according to one of claims 5 to 7,
wherein the patient information comprises at least one entry from a patient file and/or comprises a diagnostic task,
wherein the medical image is associated with a set of attributes,
wherein the method step of determining (DET-3) the set of observables comprises the following method step:
- applying (APP-3) a linguistic analysis function to the at least one entry from the patient file and/or to the diagnostic task,
wherein at least one keyword is determined;
- comparing (COMP) the at least one keyword with the set of attributes,
wherein in the event of a positive comparison, the keyword is adopted in the set of observables.

9. Method according to one of claims 5 to 8,
also comprising the following method steps:
- providing (PROV-4) the set of observables;
- receiving (REC-3) a user input relating to the set of observables,
wherein the determining of the anatomical region takes into account said user input.

10. Method according to claim 9,
wherein the user input relating to the set of observables comprises prioritising and/or deleting and/or selecting the observables.

11. Method according to one of the preceding claims,
also comprising the following method steps:
- determining (DET-4) a search profile for similar patients on the basis of the patient information and/or the medical image;
- determining (DET-5) at least one similar patient from a plurality of reference patients by applying the search profile in a medical information system in which the various reference patients are filed;
- providing (PROV-5) information relating to the at least one similar patient.

12. Method according to claim 11,
wherein the information relating to the at least one similar patient comprises a diagnosis of the similar patient,
wherein the method step of providing (PROV-5) information relating to the similar patient comprises the following method step:
- providing (PROV-6) the diagnosis of the similar patient.

13. Method according to one of claims 11 or 12,
wherein the information relating to the at least one similar patient comprises a medical image of the similar patient,
wherein the method also comprises the following method steps:
- applying (APP-4) the selected tool to the medical image of the similar patient,
wherein an edited medical image of the similar patient is determined;
- providing (PROV-7) the edited medical image of the similar patient.

14. System (SYS) for providing an edited medical image, comprising an interface (SYS.IF) and a computing unit (SYS.CU),
wherein the interface (SYS.IF) and the computing unit (SYS.CU) are designed to perform the following method steps:
- receiving (REC-1) a medical image,
wherein the medical image depicts an examination region of a patient and wherein the medical image is a two-dimensional X-ray image, an ultrasound image, a computed tomography (CT) image, a magnetic resonance tomography (MRT) image, a positron emission tomography (PET) image, or a single photon emission computed tomography (SPECT) image,
- receiving (REC-2) patient information of the patient;
- determining (DET-1) an anatomical region on the basis of the medical image and the patient information,
wherein the examination region comprises said anatomical region and wherein the anatomical region relates to an organ structure, or to a body part that comprises an abnormal region or an abnormal structure,
- selecting (SEL) a tool on the basis of the anatomical region,
wherein the tool is designed to edit anatomical region depicted in the medical image;
- applying (APP-1) the selected tool to the medical image,
wherein an edited medical image is determined;
- providing (PROV-1) the edited medical image.

15. Computer program product having a computer program, which can be loaded directly into a memory (SYS.CU) of a system (SYS), and which contains program segments in order to perform all the steps of the method according to one of claims 1 to 13 when the program segments are executed by the system (SYS).

16. Computer-readable storage medium, on which are stored program segments which can be read and executed by a system (SYS) in order to perform all the steps of the method according to one of claims 1 to 13 when the program segments are executed by the system (SYS).

## Revendications

1. Procédé mis en œuvre par ordinateur pour disposer d'une image médicale traitée,
comprenant les stades de procédé suivants :
- réception (REC-1) d'une image médicale,
dans lequel l'image médicale reproduit une zone en examen d'un patient et dans lequel l'image médicale est une image par rayons X en deux dimensions, une image par ultrasons, une image de tomodensitométrie assistée par ordinateur (CT), une image de tomodensitométrie par résonance magnétique (MRT), une image de tomodensitométrie par émission de positrons (PET) ou une image de tomodensitométrie assistée par ordinateur à émission de photons individuels (SPECT),
- réception (REC-2) d'informations du patient,
- détermination (DET-1) d'une zone anatomique sur la base de l'image médicale et des informations du patient, dans lequel la zone en examen comprend la zone anatomique et dans lequel la zone anatomique se rapporte à une structure organique ou à une partie du corps, qui comprend une zone remarquable ou une structure remarquable,
- choix (SEL) d'un outil sur la base de la zone anatomique,
dans lequel l'outil est constitué pour traiter la zone anatomique représentée dans l'image médicale,
- application (APP-1) de l'outil choisi à l'image médicale,
- mise (PROV-1) à disposition de l'image médicale traitée.

2. Procédé suivant la revendication 1,
dans lequel les informations du patient comprennent des entrées se composant d'un acte du patient et/ou d'au moins une autre image médicale du patient différente de l'image médicale et/ou d'une tâche de constatation par rapport à l'image médicale.

3. Procédé suivant la revendication 2,
comprenant en outre les stades de procédé suivants :
- détermination (DET-2), à partir des informations du patient, d'autres images médicales du patient, qui représentent la zone anatomique,
- mise (PROV-2) à disposition des autres images médicales déterminées.

4. Procédé suivant la revendication 3,
comprenant en outre le stade de procédé suivant :
- application (APP-2) de l'outil choisi aux autres images médicales déterminées, qui représentent la zone anatomique,
- mise (PROV-3) à disposition des autres images médicales traitées.

5. Procédé suivant l'une des revendications précédentes,
dans lequel le stade de procédé de la détermination (DET-1) de la zone anatomique comprend le stade de procédé suivant :
- détermination (DET-3) d'un ensemble d'observables sur la base de l'image médicale et des informations du patient, la détermination (DET-1) de la zone anatomique reposant sur l'ensemble d'observables.

6. Procédé suivant la revendication 5,
dans lequel un observable de l'ensemble d'observables est un observable interne au système ou une entrée dans un ensemble de propriétés affecté à l'image médicale ou une partie du corps suivant une constatation, suivant les informations du patient ou une valeur de laboratoire remarquable suivant des informations du patient ou une partie du corps, dont, suivant les informations du patient, un échantillon de tissu a été prélevé, ou un diagnostic suspecté ou une constatation suivant les informations du patient ou un mot clé dans une tâche de constatation pour l'image médicale.

7. Procédé suivant l'une des revendications 5 ou 6,
dans lequel, dans le stade de procédé de la détermination (DET-1) de la zone anatomique, on détermine la zone anatomique au moyen d'une identification de modèles parmi une pluralité de zones anatomiques,
dans lequel on affecte à chaque zone anatomique de la pluralité de zones anatomiques un ensemble d'observables typiques,
dans lequel on détermine parmi la pluralité de zones anatomiques au moyen de l'identification de modèles la zone anatomique, pour laquelle l'ensemble affecté d'observables typiques coïncide au mieux avec l'ensemble d'observables déterminé sur la base de l'image médicale et des informations du patient.

8. Procédé suivant l'une des revendications 5 à 7,
dans lequel les informations du patient comprennent au moins une entrée composée d'un acte du patient et/ou d'une tâche de constatation, dans lequel on affecte à l'image médicale un ensemble de propriétés,
dans lequel le stade de procédé de la détermination (DET-3) de l'ensemble d'observables comprend le stade de procédé suivant :
- application (APP-3) d'une fonction d'analyse linguistique à la au moins une entrée se composant de l'acte du patient et/ou de la tâche de constatation,
dans lequel on détermine au moins un mot qui porte,
- alignement (COMP) du au moins un mot qui porte avec l'ensemble de propriétés,
dans lequel si l'alignement est positif, on prend le mot qui porte dans l'ensemble d'observables.

9. Procédé suivant l'une des revendications 5 à 8, comprenant en outre les stades de procédé suivants :
- mise (PROV-4) à disposition de l'ensemble d'observables,
- réception (REC-3) d'une entrée utile concernant l'ensemble d'observables,
dans lequel on prend en compte l'entrée utile, lors de la détermination de la zone anatomique.

10. Procédé suivant la revendication 9,
dans lequel l'entrée utile en ce qui concerne l'ensemble d'observables comprend une priorisation et/ou un effacement et/ou un choix des observables.

11. Procédé suivant l'une des revendications précédentes, comprenant en outre les stades de procédé suivants :
- détermination (DET-4) d'un profil de recherche pour des patients analogues sur la base des informations du patient et/ou de l'image médicale,
- détermination (DET-5) d'au moins un patient semblable parmi plusieurs patients de référence par application du profil de recherche dans un système d'information médicale, dans lequel sont mis les divers patients de référence,
- mise (PROV-5) à disposition d'une information en relation avec le au moins un patient semblable.

12. Procédé suivant la revendication 11,
dans lequel l'information en relation avec le au moins un patient semblable comprend un diagnostic du patient semblable,
dans lequel le stade de procédé de la mise (PROV-5) à disposition d'une information en relation avec le patient semblable comprend le stade de procédé suivant :
- mise (PROV-6) à disposition du diagnostic du patient semblable.

13. Procédé suivant l'une des revendications 11 ou 12,
dans lequel l'information en relation avec le au moins un patient semblable comprend une image médicale du patient semblable,
dans lequel le procédé comprend en outre des stades de procédé suivants :
- application (APP-4) de l'outil choisi à l'image médicale du patient semblable,
dans lequel on détermine l'image médicale traitée du patient semblable,
- mise (PROV-7) à disposition de l'image médicale traitée du patient semblable.

14. Système (SYS) de mise à disposition d'une image médicale traitée,
comprenant une interface (SYS.IF) et une unité (SYS.CU) informatique,
dans lequel l'interface (SYS.IF) et l'unité (SYS.CU) informatique sont constituées pour effectuer les stades de procédé suivants :
- réception (REC-1) d'une image médicale,
dans lequel l'image médicale reproduit une zone en examen d'un patient et dans lequel l'image médicale est une image par rayons X en deux dimensions, une image par ultrasons, une image de tomodensitométrie assistée par ordinateur (CT), une image de tomodensitométrie par résonance magnétique (MRT), une image de tomodensitométrie par émission de positrons (PET) ou une image de tomodensitométrie assistée par ordinateur à émission de photons individuels (SPECT),
- réception (REC-2) d'informations du patient,
- détermination (DET-1) d'une zone anatomique sur la base de l'image médicale et des informations du patient, dans lequel la zone en examen comprend la zone anatomique et dans lequel la zone anatomique se rapporte à une structure organique ou à une partie du corps, qui comprend une zone remarquable ou une structure remarquable,
- choix (SEL) d'un outil sur la base de la zone anatomique,
dans lequel l'outil est constitué pour traiter la zone anatomique représentée dans l'image médicale,
- application (APP-1) de l'outil choisi à l'image médicale,
- mise (PROV-1) à disposition de l'image médicale traitée.

15. Produit de programme d'ordinateur comprenant un programme d'ordinateur, qui peut être chargé directement dans la mémoire (SYS.CU) d'un système (SYS), comprenant des parties de programme pour exécuter tous les stades du procédé suivant l'une des revendications 1 à 13, lorsque les parties du programme sont exécutées par le système (SYS).

16. Support de mémoire déchiffrable par ordinateur, sur lequel des parties de programme pouvant être déchiffrées et exécutées par un système, sont mises en mémoire pour exécuter tous les stades du procédé suivant l'une des revendications 1 à 13, lorsque les parties du programme sont exécutées par le système (SYS).
